# EUROPEAN PATENT SPECIFICATION

(11) **EP 4 083 632 B1**
(45) Date of publication and mention of the grant of the patent: **09.09.2026**
(21) Application number: 22170397.8
(22) Date of filing: 28.04.2022
(51) Int. Cl.: G01N 35/00, G01N 35/10, G06K 7/10, G06K 19/073

(54) **SAMPLE ANALYZER AND REAGENT INFORMATION READING METHOD APPLIED TO SAID SAMPLE ANALYZER**
PROBENANALYSATOR UND VERFAHREN ZUM LESEN VON REAGENZINFORMATIONEN, DAS AUF DEN PROBENANALYSATOR ANGEWENDET WIRD
ANALYSEUR D'ÉCHANTILLONS ET PROCÉDÉ DE LECTURE D'INFORMATIONS SUR LES RÉACTIFS APPLIQUÉ AU-DIT ANALYSEUR D'ÉCHANTILLONS

(30) Priority: 28.04.2021 CN 202110467110
(43) Date of publication of application: 02.11.2022
(73) Proprietor: Shenzhen Mindray Bio-Medical Electronics Co., Ltd., Shenzhen, Guangdong 518057 (CN)
(72) Inventor: DU, Xiansuan, Shenzhen, 518057 (CN); JIA, Zhenjie, Shenzhen, 518057 (CN)
(74) Representative: KIPA AB

(56) References cited:
- EP-A2- 2 372 369
- WO-A1-2020/158039
- WO-A2-2008/014117
- GB-A- 2 568 915

## Description

### TECHNICAL FIELD

The disclosure relates to the technical field of medical instruments, and in particular to a sample analyzer and a reagent information reading method.

### BACKGROUND

Sample analyzers are widely used in clinical testing, and existing sample analyzers mainly include a glycated hemoglobin analyzer, a blood cell analyzer, a whole blood CRP analyzer, etc. During blood analysis, a chemical reagent is required to support analysis of a blood sample. When the chemical reagent is used up, a user needs to replace it with a new reagent to support a sample analyzer in continuing analysis. Generally, the chemical reagent used by the sample analyzer is stored in a reagent supply device of the sample analyzer. The reagent supply device reads an information label provided on a reagent container by means of a corresponding information read/write device, to obtain information about a reagent stored in the reagent container.

However, when a plurality of reagent containers are placed in a same reagent supply device, a plurality of corresponding information labels may all be located in an electromagnetic field of a same information read/write device, resulting in a problem of adjacent channel crosstalk. Consequently, the information label on a corresponding reagent container cannot be accurately read, resulting in reagent information errors.

European patent application EP2372369A2 has disclosed a sample analyzer, comprising a reagent container set section for setting a reagent container, a reader/writer configured to read out an information from the recording medium attached to the reagent container set in the reagent container set section and configured to write an information on the recording medium, a writing instruction section configured to issue an instruction to write the information on the recording medium, and a controller configured to control the reader/writer to write the information on the recording medium attached to the reagent container set in the reagent container set section if the kind information read out from the recording medium indicates the specific reagent and the writing instruction section has issued the writing instruction. In this patent application, however, each antenna is attached to the side portion of each reagent container installation section. When a reagent container is positioned at the setting position, the RFID tag of the reagent container is disposed next to the antennas in the reagent container installation section in which the reagent container is installed. Accordingly, the reflected wave from the RFID tag of the reagent container installed in the reagent container installation section is received by the nearest of the antennas (that is, the one disposed next to the tag). The reflected wave sent from the RFID tag is very weak and is not received by the other antennas other than the nearest antenna.

### SUMMARY

A main objective of embodiments of the disclosure is to provide a sample analyzer, a reagent supply device and a reagent information reading method, which are intended to solve the problem of adjacent channel crosstalk that may occur when a plurality of reagent containers are placed in a reagent storage device, so as to accurately read information label on the corresponding reagent containers.

The invention is set out in the appended set of claims.

According to a first aspect, an embodiment of the disclosure provides a sample analyzer according to claim 1.

According to a second aspect, an embodiment of the disclosure further provides a reagent information reading method according to claim 14.

Preferred embodiments are defined in the dependent claims.

In the disclosure, when the information read/write device interacts with the information storage component in the target reagent position by means of the signal transmission circuit, the electromagnetic field shielding circuit generates a counter electromagnetic field, and the counter electromagnetic field is used to weaken an electromagnetic field transmitted from the emission electromagnetic field to a non-target reagent position (for example, a reagent position adjacent to the target reagent position), so that the information read/write device cannot establish a communication connection with the information storage component in the non-target reagent position, thereby solving the problem of adjacent channel crosstalk that occurs when reading information. In this way, the signal transmission circuit corresponding to the target reagent position can accurately read an information label on a corresponding reagent bottle to accurately obtain reagent information of the reagent stored in the reagent container located in the target reagent position.

### BRIEF DESCRIPTION OF THE DRAWINGS

In order to more clearly describe the technical solutions in the embodiments of the disclosure, the drawings required for describing the embodiments will be briefly described below. Apparently, the drawings in the following description show some of the embodiments of the disclosure, and persons of ordinary skill in the art may still derive other drawings from these drawings without creative efforts.
FIG. 1 is a block diagram of a schematic structure of a sample analyzer according to an embodiment of the disclosure;
FIG. 2 is a block diagram of a schematic structure of a reagent supply device according to an aspect of an embodiment of the disclosure;
FIG. 3 is a block diagram of another schematic structure of a reagent supply device according to an aspect of an embodiment of the disclosure;
FIGS. 4A to 4C are schematic diagrams of variants of the reagent supply device according aspects of embodiments of the disclosure;
FIGS. 5A to 5C are schematic diagrams of structures of an arrangement relationship between a reagent position of a reagent supply device and an electromagnetic field shielding circuit according to an aspect of an embodiment of the disclosure;
FIG. 5D is a schematic diagram of a perspective structure of a reagent placement compartment of a reagent supply device according to an aspect of an embodiment of the disclosure;
FIG. 5E is a schematic diagram of an application scenario of a reagent placement compartment according to an aspect of an embodiment of the disclosure;
FIG. 5F is a schematic diagram of an exploded structure of a reagent placement compartment according to an aspect of an embodiment of the disclosure;
FIG. 6 to 15 are block diagrams of schematic structures of different implementations of a reagent supply device according to aspects of embodiments of the disclosure; and
FIG. 16 is a flowchart of steps of a reagent information reading method according to an embodiment of the disclosure.

### DESCRIPTION OF THE PREFERRED EMBODIMENTS

The technical solutions of the embodiments of the disclosure will be described below clearly and comprehensively in conjunction with accompanying drawings of the embodiments of the disclosure. Apparently, the embodiments described are some of, rather than all of, the embodiments of the disclosure.

In the description of the disclosure, the terms "mount", "engage", and "connect" should be interpreted in a broad sense unless explicitly defined and limited otherwise, which, for example, may mean a fixed connection, a detachable connection, or an integral connection; may mean a mechanical connection or an electrical connection; and may mean a direct connection, an indirect connection by means of an intermediary, or internal communication between two elements. For persons of ordinary skill in the art, specific meanings of the foregoing terms in the disclosure may be understood based on specific situations.

Some implementations of the disclosure are described in detail below in conjunction with the drawings. In the case of no conflict, the embodiments and the features in the embodiments described below can be combined with each other.

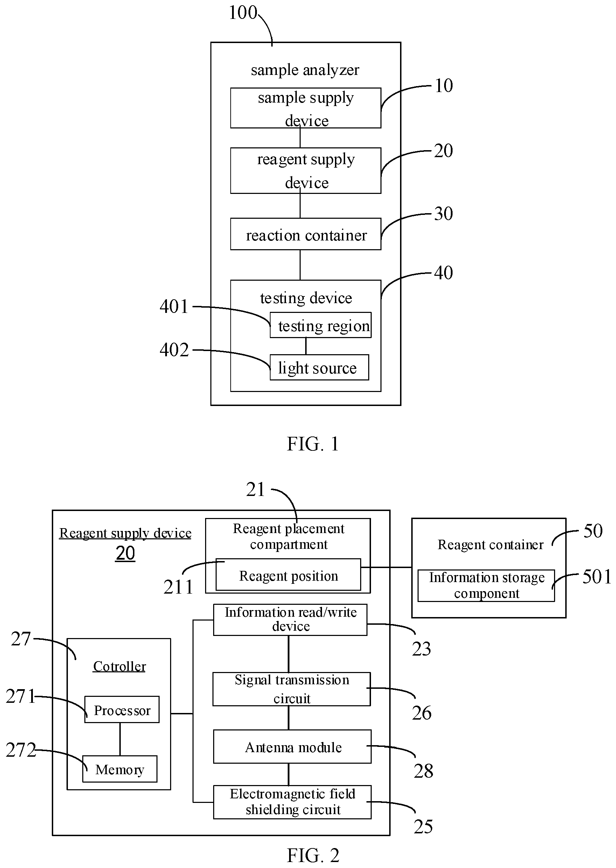

Referring to FIGS. 1 and 2, the disclosure provides a sample analyzer 100 for analyzing a sample to be tested, to obtain a corresponding analysis result. The sample to be tested includes but is not limited to a blood sample.

The sample analyzer 100 includes a sample supply device 10, a reagent supply device 20, a reaction container 30, and a testing device 40. The sample supply device 10 is configured to supply a blood sample to be tested; the reagent supply device 20 is configured to supply a reagent for reacting with the blood sample to be tested; the reaction container 30 is configured to receive the blood sample to be tested that is supplied by the sample supply device 10 and the reagent that is supplied by the reagent supply device 20, so that the blood sample to be tested and the reagent react to form a mixed sample solution; the testing device 40 includes a testing region 401 made of a light-transmitting material and a light source 402 corresponding to the testing region 401, the light source 402 being configured to irradiate the mixed sample solution flowing through the testing region 401, so as to measure content of a specific protein in the mixed sample solution. For example, the light source 402 of the testing device 40 irradiates the mixed sample solution flowing through the testing region 401, and the content of the specific protein in the mixed sample solution is measured by monitoring a change rate of transmitted and/or scattered light signals and using a preset calculation method, wherein the reagent for reacting with the blood sample to be tested includes, but is not limited to, a latex reagent, a diluent, a staining agent, or a hemolytic agent. The content of the specific protein includes, but is not limited to, C-reactive protein (CRP), serum amyloid A (SAA), immunoglobulins C3/C4, antihemolytic streptococcus O (ASO), and rheumatoid factor (RF).

As shown in FIG. 2, the reagent supply device 20 is provided with a reagent placement compartment 21, an information read/write device 23, an electromagnetic field shielding circuit 25, a signal transmission circuit 26, a controller 27, and an antenna module 28. The reagent placement compartment 21 is provided with at least two reagent positions 211, wherein the at least two reagent positions 211 are respectively used for placing a reagent container 50 containing a reagent, wherein the reagent container 50 is provided with an information storage component 501 storing reagent information. The reagent information includes, but is not limited to, any one or a combination of two or more of reagent component, expiration date, production date, lot number, manufacturer, reagent capacity, and reagent volume. The information storage component 501 includes, but is not limited to, a radio frequency identification (RFID) label. Reagents stored in corresponding reagent containers 50 placed in the at least two reagent positions 211 may be the same or may be different, which are not limited herein. For example, a corresponding reagent container 50 placed in at least one of the reagent positions 211 stores a latex reagent, and a corresponding reagent container 50 placed in another reagent position 211 stores any of a diluent, a staining agent, and a hemolytic agent. Alternatively, the corresponding reagent containers 50 placed in the at least two reagent positions 211 store a same reagent, for example, any of a latex reagent, a diluent, a staining agent, and a hemolytic agent.

The signal transmission circuit 26 is capable of being connected to the antenna module 28 to transmit an emission electromagnetic field. The information read/write device 23 is connected to the signal transmission circuit 26 and is configured to perform signal interaction with the information storage component 501 of the reagent container 50 in a target reagent position. The electromagnetic field shielding circuit 25 is configured to generate a counter electromagnetic field, so as to weaken the emission electromagnetic field transmitted to a non-target reagent position.

As an example,, the signal transmission circuit 26 is disposed corresponding to the reagent positions 211 and is connected to the antenna module 28, so as to transmit an emission electromagnetic field to the information storage component 501 in the target reagent position 211 in cooperation with the antenna module 28. The information read/write device 23 is connected to the signal transmission circuit 26, to perform signal interaction with the information storage component 501 of the reagent container 50 in the target reagent position, and read or write information from or to the information storage component 501, so as to obtain reagent information stored in the information storage component 501 or write corresponding reagent information to the information storage component 501. The electromagnetic field shielding circuit 25 is configured to generate a counter electromagnetic field, so as to weaken an electromagnetic field transmitted from the emission electromagnetic field generated by the information read/write device 23 in cooperation with the signal transmission circuit 26 and the antenna module 28 to another non-target reagent position. In this example, the electromagnetic field shielding circuit 25 may generate the counter electromagnetic field by connecting to the antenna module 28, or the electromagnetic field shielding circuit 25 may include an antenna module to generate the counter electromagnetic field.

The controller 27 is electrically connected to the sample supply device 10, the reagent supply device 20, and the testing device 40, and is configured to control the information read/write device 23 to transmit the emission electromagnetic field by means of the signal transmission circuit 26 and the antenna module 28 corresponding to a target reagent position, so that the signal transmission circuit 26 corresponding to the target reagent position performs signal interaction with the information storage component 501 in the target reagent position. In this way, the information read/write device 23 reads or writes information from or to the information storage component 501 placed in the target reagent position by means of the signal transmission circuit 26 and the antenna module 28.

In addition, the controller 27 is further configured to control the electromagnetic field shielding circuit 25 to generate the counter electromagnetic field, so as to weaken an electromagnetic field transmitted from the emission electromagnetic field to another non-target reagent position, so that the electromagnetic field emitted and transmitted to the another non-target reagent position by the signal transmission circuit 26 corresponding to the target reagent position by mean of the antenna module 28 is not enough to activate the corresponding information storage component 501 disposed in the another non-target reagent position, which avoids a problem of signal crosstalk between adjacent reagent information reading channels that is caused by the corresponding information read/write device 23 obtaining reagent information corresponding to the another non-target reagent position due to interaction between the signal transmission circuit 26 and the information storage component 501 in the another reagent position. It is ensured that when a plurality of reagent containers 50 are placed in a plurality of corresponding reagent positions 211 in a same reagent supply device 20, the information read/write device 23 can accurately read reagent information in the corresponding information storage component 501 placed in the target reagent position. The target reagent position is a reagent position 211 in which a reagent container 50 to be subjected to information reading is placed.

In this implementation, by providing the electromagnetic field shielding circuit 25 that can generate a counter electromagnetic field, when reagent information corresponding to a reagent stored in the reagent container 50 placed in the target reagent position needs to be read, the controller is used to control the information read/write device 23 to generate an emission electromagnetic field by means of the signal transmission circuit 26 and the antenna module 28, so as to interact with the information storage component 501 of the reagent container 50 placed in the target reagent position and control the electromagnetic field shielding circuit 25 to generate the counter electromagnetic field for weakening the electromagnetic field emitted by the signal transmission circuit 26, so that the electromagnetic field emitted by the signal transmission circuit 26 is not enough to activate the information storage component 501 in the another non-target reagent position and establish a communication connection with the information storage component 501 in the another non-target reagent position for interaction, thereby ensuring that the information read/write device 23 can accurately read the reagent information in the corresponding information storage component 501 placed in the target reagent position.

As shown in FIG. 2, in some implementations, the controller 27 includes a processor 271 and a memory 272. The memory 272 may be a volatile memory (volatile memory), such as a random access memory (RAM); or a non-volatile memory (non-volatile memory), such as a read-only memory (ROM), a flash memory (flash memory), a hard disk drive (HDD), or a solid-state drive (SSD); or a combination of the above types of memories. The memory 272 is configured to store a computer program, and may provide instructions and data to the processor 271.

The processor 271 may be a central processing unit (CPU), or the processor may be another general-purpose processor, a digital signal processor (DSP), an application-specific integrated circuit (ASIC), a field-programmable gate array (FPGA), or another programmable logic device, a discrete gate or transistor logic device, a discrete hardware component, or the like. The general-purpose processor may be a microprocessor, or the processor may be any conventional processor, etc.

The processor 271 can call the computer program stored in the memory 272, to control the information read/write device 23 to transmit the emission electromagnetic field by means of the signal transmission circuit 26 and the antenna module 28 corresponding to the target reagent position, so that the information read/write device 23 corresponding to the target reagent position performs signal interaction with the information storage component 501 in the target reagent position; and control the electromagnetic field shielding circuit 25 to generate the counter electromagnetic field, so as to weaken an electromagnetic field transmitted from the emission electromagnetic field to another non-target reagent position.

In a specific example, the at least two reagent positions 211 comprise a first reagent position 211a and a second reagent position 211b which are independent of each other. The controller 27 may be configured to: control the information read/write device 23 to transmit the emission electromagnetic field by means of the signal transmission circuit 26 and the antenna module 28, so that the information read/write device 23 performs signal interaction with the information storage component of the reagent container in the first reagent position 211a (namely the target reagent position) of the at least two reagent positions 211; and control the electromagnetic field shielding circuit 25 to generate the counter electromagnetic field, so as to weaken an electromagnetic field transmitted from the emission electromagnetic field to the second reagent position 211b (namely the non-target reagent position) of the at least two reagent positions 211.

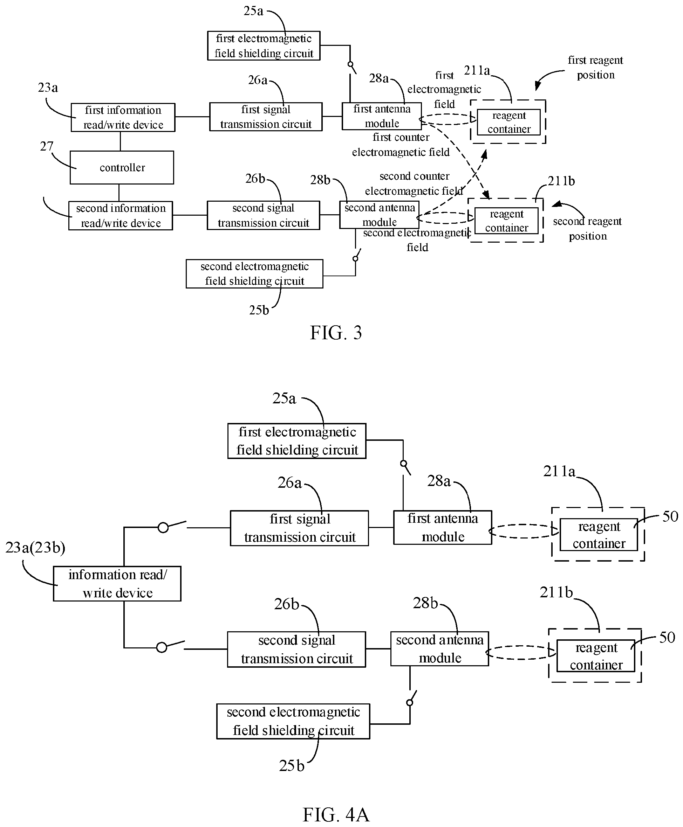

In some implementations, as shown in FIG. 3, the antenna module 28 includes a first antenna module 28a corresponding to the first reagent position 211a and a second antenna module 28b corresponding to the second reagent position 211b; the signal transmission circuit 26 includes a first signal transmission circuit 26a corresponding to the first reagent position 211a and a second signal transmission circuit 26b corresponding to the second reagent position 211b; the information read/write device 23 includes a first information read/write device 23a corresponding to the first reagent position 211a and a second information read/write 23b device corresponding to the second reagent position 211b, and the electromagnetic field shielding circuit 25 includes a first electromagnetic field shielding circuit 25a corresponding to the first reagent position 211a and a second electromagnetic field shielding circuit 25b corresponding to the second reagent position 211b. That is, the reagent supply device 20 includes the first antenna module 28a, the first signal transmission circuit 26a, the first information read/write device 23a and the first electromagnetic field shielding circuit 25a which correspond to the first reagent position 211a, and includes the second antenna module 28b, the second signal transmission circuit 26b, the second information read/write device 23b and the second electromagnetic field shielding circuit 25b which correspond to the second reagent position 211b.

In the implementations shown in FIG. 3, the first information read/write device 23a is capable of being connected to the first antenna module 28a via the first signal transmission circuit 26a, so as to transmit a first electromagnetic field to the information storage component 501 of the reagent container 50 in the first reagent position 211a and perform signal interaction with the information storage component 501 of the reagent container 50 in the first reagent position 211a, and the first electromagnetic field shielding circuit 25a is capable of being connected to the first antenna module 28a to generate a first counter electromagnetic field. The second information read/write device 23b is capable of being connected to the second antenna module 28b via the second signal transmission circuit 26b, so as to transmit a second electromagnetic field to the information storage component 501 of the reagent container 50 in the second reagent position 211b and perform signal interaction with the information storage component 501 of the reagent container 50 in the second reagent position 211b, and the second electromagnetic field shielding circuit 25b is capable of being connected to the second antenna module 28b to generate a second counter electromagnetic field.

In the implementations shown in FIG. 3, when the first reagent position 211a is the target reagent position, the controller 27 is configured to control the first information read/write device 23a to transmit the first electromagnetic field by means of the first signal transmission circuit 26a and the first antenna module 28a to the information storage component 501 of the reagent container 50 in the first reagent position 211a, so that the first information read/write device 23a performs signal interaction with the information storage component 501 of the reagent container 50 in the first reagent position 211a; and control the second electromagnetic field shielding circuit 25b to be connected to the second antenna module 28b to generate the second counter electromagnetic field, so as to weaken an electromagnetic field transmitted from the first electromagnetic field to the second reagent position 211b, so that the first electromagnetic field is not enough to activate the information storage component 501 in the second reagent position 211b and establish a communication connection with the information storage component 501 in the second reagent position 211b for interaction, thereby ensuring that the information read/write device 23a can accurately read the reagent information in the information storage component 501 placed in the first reagent position 211a. Alternatively, when the second reagent position 211b is the target reagent position, the controller 27 is configured to control the second information read/write device 23b to transmit the second electromagnetic field by means of the second signal transmission circuit 26b and the second antenna module 28b to the information storage component 502 of the reagent container 50 in the second reagent position 211b, so that the second information read/write device 23b performs signal interaction with the information storage component 501 of the reagent container 50 in the second reagent position 211b; and control the first electromagnetic field shielding circuit 25a to be connected to the first antenna module 28a to generate the first counter electromagnetic field, so as to weaken an electromagnetic field transmitted from the second electromagnetic field to the first reagent position 211a, so that the second electromagnetic field is not enough to activate the information storage component 501 in the first reagent position 211a and establish a communication connection with the information storage component 501 in the first reagent position 211a for interaction, thereby ensuring that the information read/write device 23b can accurately read the reagent information in the information storage component 501 placed in the second reagent position 211b.

In a specific example, the first antenna module 28a includes a first working mode for transmitting the first electromagnetic field, and a second working mode for generating the first counter electromagnetic field, and the controller 27 is further configured to control the first antenna module 28a to work in the first working mode or in the second working mode. When the first antenna module 28a is in the first working mode, the first antenna module 28a is connected to the first signal transmission circuit 26a and then connected to the first information read/write device 23a, so that the first information read/write device 23a transmits the first electromagnetic field by means of the first signal transmission circuit 26a and the first antenna module 28a to the information storage component 501 of the reagent container 50 in the first reagent position 211a, so that the first information read/write device 23a performs signal interaction with the information storage component 501 of the reagent container 50 in the first reagent position 211a; and the first antenna module 28a is disconnected from the first electromagnetic field shielding circuit 25a. When the first antenna module 28a is in the second working mode, the first antenna module 28a is connected to the first electromagnetic field shielding circuit 25a to generate the first counter electromagnetic field, and the first antenna module 28a is disconnected from the first signal transmission circuit 26a. Also, the second antenna module 28b includes a first working mode for transmitting the second electromagnetic field, and a second working mode for generating the second counter electromagnetic field, and the controller 27 is further configured to control the second antenna module 28b to work in the first working mode or in the second working mode. When the second antenna module 28b is in the first working mode, the second antenna module 28b is connected to the second signal transmission circuit 26b and then connected to the second information read/write device 23b, so that the second information read/write device 23b transmits the second electromagnetic field by means of the second signal transmission circuit 26b and the second antenna module 28b to the information storage component 501 of the reagent container 50 in the second reagent position 211b, so that the second information read/write device 23b performs signal interaction with the information storage component 501 of the reagent container 50 in the second reagent position 211b; and the second antenna module 28b is disconnected from the second electromagnetic field shielding circuit 25b. When the second antenna module 28b is in the second working mode, the second antenna module 28b is connected to the second electromagnetic field shielding circuit 25b to generate the second counter electromagnetic field, and the second antenna module 28b is disconnected from the second signal transmission circuit 26b. In the example of the disclosure, when the first antenna module 28a is in its first working mode, the second antenna module 28b is in its second working mode; and when the second antenna module 28b is in its first working mode, the first antenna module 28a is in its second working mode.

In some implementations, a direction of the counter electromagnetic field generated by the electromagnetic field shielding circuit 25 is opposite to that of the emission electromagnetic field transmitted by the information read/write device 23 via the signal transmission circuit 26 and the antenna module 28 corresponding to the target reagent position.

In an example, when the first reagent position 211a is the target reagent position, a field direction of the second counter electromagnetic field generated by the second electromagnetic field shielding circuit 25b is opposite to that of the first electromagnetic field transmitted by the first information read/write device 23a via the first signal transmission circuit 26a and the first antenna module 28a. or, when the second reagent position 211b is the target reagent position, a field direction of the first counter electromagnetic field generated by the first electromagnetic field shielding circuit 25a is opposite to that of the second electromagnetic field transmitted by the second information read/write device 23b via the second signal transmission circuit 26b and the second antenna module 28b.

Because the direction of the counter electromagnetic field is opposite to that of the emission electromagnetic field, the counter electromagnetic field generated by the electromagnetic field shielding circuit 25 can weaken an electromagnetic field transmitted from the emission electromagnetic field to another reagent position (i.e. a non-target reagent position), so that the electromagnetic field transmitted from the emission electromagnetic field to the another reagent position is not enough to activate the information storage component 501 in the another reagent position and establish a communication connection with the information storage component 501 in the another reagent position for interaction, thereby ensuring that the information read/write device 23 can interact only with the information storage component 501 in the target reagent position to obtain reagent information of the reagent container 50 placed in the corresponding target reagent position.

In terms of adjusting the direction of the counter electromagnetic field to be opposite to that of the emission electromagnetic field, a current of the electromagnetic field shielding circuit 25 may be adjusted, so that a direction of the current of the electromagnetic field shielding circuit 25 is opposite to that of a current of the signal transmission circuit 26 corresponding to the target reagent position, such that the direction of the counter electromagnetic field is opposite to that of the emission electromagnetic field.

In an example, when the first reagent position 211a is the target reagent position, controlling the second electromagnetic field shielding circuit 25b to generate the second counter electromagnetic field, so as to weaken an electromagnetic field transmitted from the first electromagnetic field to the second reagent position 211b may include: adjusting a current of the second electromagnetic field shielding circuit 25b, so that a direction of the current of the electromagnetic field shielding circuit 25b is opposite to that of the current of the first signal transmission circuit 26a. or, when the second reagent position 211b is the target reagent position, controlling the first electromagnetic field shielding circuit 25a to generate the first counter electromagnetic field, so as to weaken an electromagnetic field transmitted from the second electromagnetic field to the first reagent position 211a may include: adjusting a current of the first electromagnetic field shielding circuit 25a, so that a direction of the current of the electromagnetic field shielding circuit 25a is opposite to that of the current of the second signal transmission circuit 26b.

In some implementations, a direction of the counter electromagnetic field generated by the electromagnetic field shielding circuit 25 is opposite to that of the emission electromagnetic field transmitted by the information read/write device 23 via the signal transmission circuit 26 and the antenna module 28 corresponding to the target reagent position, and a strength of the counter electromagnetic field is greater than or equal to a strength of the emission electromagnetic field.

In an example, when the first reagent position 211a is the target reagent position, a field strength of the second counter electromagnetic field generated by the second electromagnetic field shielding circuit 25b is greater than or equal to a field strength of the first electromagnetic field transmitted by the first information read/write device 23a via the first signal transmission circuit 26a and the first antenna module 28a. or, when the second reagent position 211b is the target reagent position, a field strength of the first counter electromagnetic field generated by the first electromagnetic field shielding circuit 25a is greater than or equal to a field strength of the second electromagnetic field transmitted by the second information read/write device 23b via the second signal transmission circuit 26b and the second antenna module 28b.

Because the direction of the counter electromagnetic field is opposite to that of the emission electromagnetic field, and the strength of the counter electromagnetic field is greater than or equal to the strength of the emission electromagnetic field, the counter electromagnetic field generated by the electromagnetic field shielding circuit 25 is enough to weaken an electromagnetic field transmitted from the emission electromagnetic field to another reagent position (i.e., a non-target reagent position), so that the electromagnetic field transmitted from the emission electromagnetic field to the another reagent position is not enough to activate the information storage component 501 in the another reagent position and establish a communication connection with the information storage component 501 in the another reagent position for interaction, thereby ensuring that the information read/write device can interact only with the information storage component 501 in the target reagent position to obtain reagent information of the reagent container 50 placed in the corresponding target reagent position.

In terms of adjusting the direction of the counter electromagnetic field to be opposite to that of the emission electromagnetic field, and the strength of the counter electromagnetic field to be greater than or equal to the strength of the emission electromagnetic field, a current of the electromagnetic field shielding circuit 25 may be adjusted, so that a direction of the current of the electromagnetic field shielding circuit 25 is opposite to that of a current of the signal transmission circuit 26 corresponding to the target reagent position, and the current of the electromagnetic field shielding circuit 25 is greater than or equal to that of the signal transmission circuit 26 corresponding to the target reagent position, such that the direction of the counter electromagnetic field is opposite to that of the emission electromagnetic field, and the strength of the counter electromagnetic field is greater than or equal to the strength of the emission electromagnetic field.

In an example, when the first reagent position 211a is the target reagent position, controlling the second electromagnetic field shielding circuit 25b to generate the second counter electromagnetic field, so as to weaken an electromagnetic field transmitted from the first electromagnetic field to the second reagent position 211b may include: adjusting a current of the second electromagnetic field shielding circuit 25b, so that a direction of the current of the electromagnetic field shielding circuit 25b is opposite to that of the current of the first signal transmission circuit 26a, and the current of the second electromagnetic field shielding circuit 25b is greater than or equal to that of the first signal transmission circuit 26a. or, when the second reagent position 211b is the target reagent position, controlling the first electromagnetic field shielding circuit 25a to generate the first counter electromagnetic field, so as to weaken an electromagnetic field transmitted from the second electromagnetic field to the first reagent position 211a may include: adjusting a current of the first electromagnetic field shielding circuit 25a, so that a direction of the current of the electromagnetic field shielding circuit 25a is opposite to that of the current of the second signal transmission circuit 26b, and the current of the first electromagnetic field shielding circuit 25a is greater than or equal to that of the second signal transmission circuit 26a.

In some implementations, the first signal transmission circuit 26a and the second signal transmission circuit 26b are independent of each other; and/or, the first electromagnetic field shielding circuit 25a and the second electromagnetic field shielding circuit 25b are independent of each other; and/or, the first information read/write device 23a and the second information read/write device 23b are independent of each other.

In some alternative or additional implementations, the first signal transmission circuit 26a and the second signal transmission circuit 26b are a same signal transmission circuit; and/or the first electromagnetic field shielding circuit 25a and the second electromagnetic field shielding circuit 25b are a same electromagnetic field shielding circuit; and/or the first information read/write device 23a and the second information read/write device 23b are a same information read/write device.

As shown in FIG. 4A, the first signal transmission circuit 26a and the second signal transmission circuit 26b are independent of each other, the first electromagnetic field shielding circuit 25a and the second electromagnetic field shielding circuit 25b are independent of each other, and the first information read/write device 23a and the second information read/write device 23b are a same information read/write device 23a (23b), wherein the information read/write device 23a (23b) is capable of being connected to the first signal transmission circuit 26a or the second signal transmission circuit 26b via a switch component.

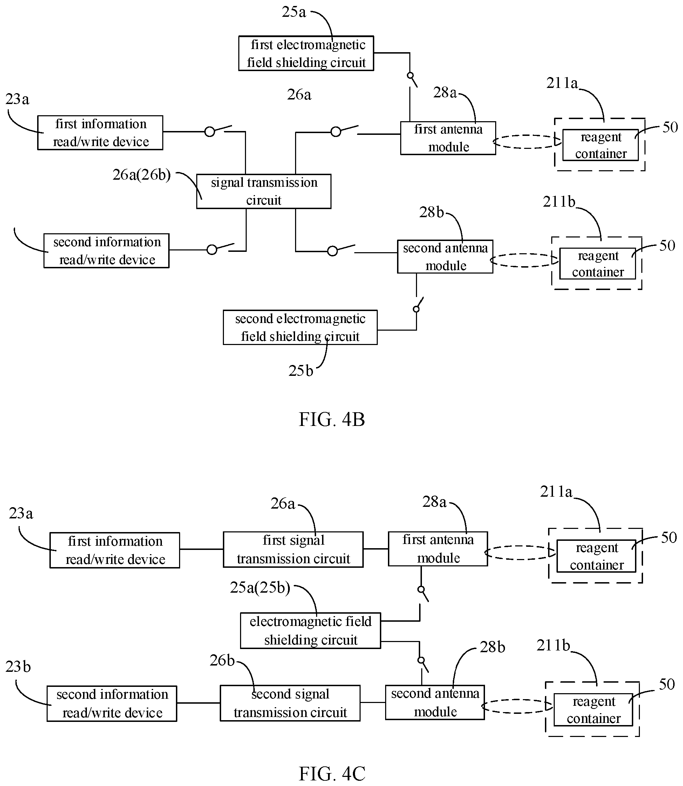

As shown in FIG. 4B, the first electromagnetic field shielding circuit 25a and the second electromagnetic field shielding circuit 25b are independent of each other, the first information read/write device 23a and the second information read/write device 23b are independent of each other, and the first signal transmission circuit 26a and the second signal transmission circuit 26b are a same signal transmission circuit 26a (26b), wherein the signal transmission circuit 26a (26b) is capable of being connected to the first information read/write device 23a and the first antenna module 28a or connected to the second read/write device 23b and the second antenna module 28b via a switch component.

As shown in FIG. 4C, the first signal transmission circuit 26a and the second signal transmission circuit 26b are independent of each other, the first information read/write device 23a and the second information read/write device 23b are independent of each other, and the first electromagnetic field shielding circuit 25a and the second electromagnetic field shielding circuit 25b are a same electromagnetic field shielding circuit 25a (25b), wherein the electromagnetic field shielding circuit 25a (25b) is capable of being connected to the first antenna module 28a or the second antenna module 28b via a switch component.

It can understand that, the opening and closing of the switch component in the embodiments of the disclosure may be controlled by the controller 27.

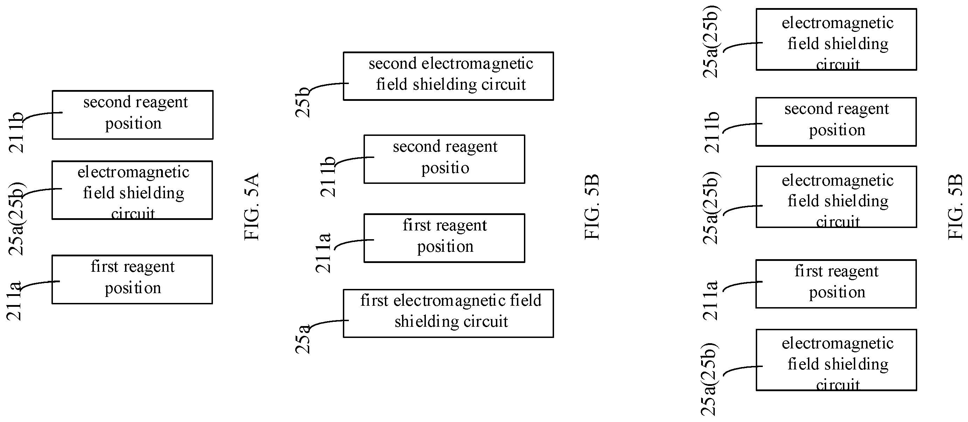

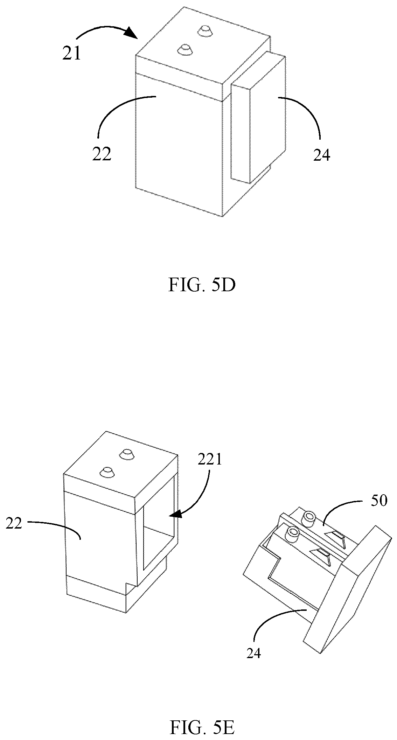

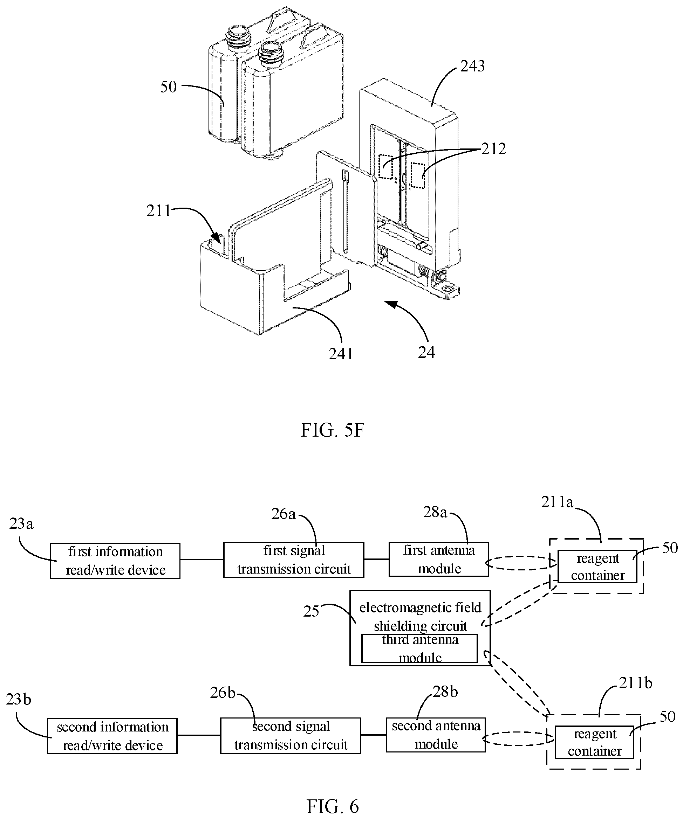

Referring to FIGS. 5A to 5C, in some implementations, the at least two reagent positions 211 are arranged in parallel according to a preset direction, and one or more electromagnetic field shielding circuits are provided. One side of one reagent position 211 and the other opposite side of another reagent position 211 are provided with electromagnetic field shielding circuits 25, or an electromagnetic field shielding circuit 25 is disposed between two adjacent reagent positions 211, or two opposite sides of each reagent position 211 are provided with electromagnetic field shielding circuits 25, to implement electromagnetic shielding for reagent information of a reagent container 50 placed in a non-target reagent position by means of the electromagnetic field shielding circuit 25.

In an example, the reagent positions 211 at least include the first reagent position 211a and the second reagent position 211b. The first reagent position 211a and the second reagent position 211b are arranged in parallel according to a preset direction, and at least the first electromagnetic field shielding circuit 25a and/or the second electromagnetic field shielding circuit 25b is provided between the first reagent position 211a and the second reagent position 211b, and preferably the first electromagnetic field shielding circuit 25a and the second electromagnetic field shielding circuit 25b are a same electromagnetic field shielding circuit, as shown in FIG. 5A.

In another example, the first reagent position 211a and the second reagent position 211b are arranged in parallel according to a preset direction, and the first electromagnetic field shielding circuit 25a is provided on one side of the first reagent position 211a opposite to the second reagent position 211b, and/or the second electromagnetic field shielding circuit 25b is provided on one side of the second reagent position 211b opposite to the first reagent position 211a, as shown in FIG. 5B.

In another example, the first reagent position 211a and the second reagent position 211b are arranged in parallel according to a preset direction, and the first electromagnetic field shielding circuit 25a and/or the second electromagnetic field shielding circuit 25b is provided between the first reagent position 211a and the second reagent position 211b, and the first electromagnetic field shielding circuit 25a is provided on one side of the first reagent position 211a opposite to the second reagent position 211b, and/or the second electromagnetic field shielding circuit 25b is provided on one side of the second reagent position 211b opposite to the first reagent position 211a, as shown in FIG. 5C.

Referring to FIGS. 5D to 5F, in some implementations,, the reagent placement compartment 21 includes a compartment body 22 and a reagent placement assembly 24 slidably disposed corresponding to the compartment body 22. The reagent placement assembly 24 is provided with at least two reagent positions 211 arranged in parallel according to a preset direction, wherein one side of one reagent position 211 and the other opposite side of another reagent position 211 are provided with electromagnetic field shielding circuits 25, or two opposite sides of each reagent position 211 are provided with electromagnetic field shielding circuits 25, or an electromagnetic field shielding circuit 25 is disposed between two adjacent reagent positions 211, to implement electromagnetic shielding for reagent information of a reagent container 50 placed in a non-target reagent position by means of the electromagnetic field shielding circuit 25. For example, the first electromagnetic field shielding circuit 25a is provided on one side of the first reagent position 211a, and the second electromagnetic field shielding circuit 25b is provided on one side of the second reagent position 211b. For another example, both sides of the first reagent position 211a are provided with the first electromagnetic field shielding circuit 25a. For yet another example, at least one of the first electromagnetic field shielding circuit 25a and the second electromagnetic field shielding circuit 25b is provided between the first reagent position 211a and the second reagent position 211b.

The compartment body 22 is formed with an accommodating cavity 221 having an opening; the reagent placement assembly 24 is in a slidable fit with the compartment body 22, and can enter or leave the accommodating cavity 221 through the opening, so as to be at least partially accommodated in the accommodating cavity 221 and cover the opening of the accommodating cavity 221, thereby storing reagent containers 50 placed in the reagent positions 211. The signal transmission circuit 26 is disposed corresponding to the reagent positions 211 and is connected to the information read/write device 23 and the antenna module 28, so as to transmit the emission electromagnetic field to the information storage component 501 in the corresponding reagent position 211, so that the information read/write device 23 can read reagent information stored in the reagent container 50 placed in the corresponding reagent position 211.

The reagent placement assembly 24 includes a bracket 241 and a compartment door module 243 connected to the bracket 241. The at least two reagent positions 211 are formed in the bracket 241. The signal transmission circuit 26 is fixed on the compartment door module 243, and is disposed corresponding to the reagent positions 211. Under the action of an external force, the bracket 241 can enter or leave the accommodating cavity 221 through the opening of the accommodating cavity 221, and can cover the opening of the accommodating cavity 221 by means of the door module 243.

The signal transmission circuit 26 may be disposed corresponding to the reagent positions 211 in the following manners: One reagent position 211 is disposed corresponding to at least two signal transmission circuits 26, or one signal transmission circuit 26 is disposed corresponding to at least two reagent positions 211, or one reagent position 211 is disposed corresponding to one signal transmission circuit 26. Preferably, the signal transmission circuits 26 and the reagent positions 211 are disposed in a one-to-one correspondence, and the parallel arrangement direction of the signal transmission circuits 26 is the same as that of the reagent positions 211, so that each signal transmission circuit 26 can better read reagent information of the reagent container 50 placed in the preset reagent position 211. For example, at least two first signal transmission circuits 26a are provided corresponding to the first reagent position 211a, and at least two second signal transmission circuits 26b are provided corresponding to the second reagent position 211b. For another example, at least one of the first signal transmission circuit 26a and the second signal transmission circuit 26b is provided corresponding to both the first reagent position 211a and the second reagent position 211b (e.g., the first signal transmission circuit 26a and the second signal transmission circuit 26b are a same signal transmission circuit). For yet another example, the first signal transmission circuit 26a is provided corresponding to the first reagent position 211a, and the second signal transmission circuit 26b is provided corresponding to the second reagent position 211b (i.e., the first signal transmission circuit 26a and the second signal transmission circuit 26b are independent of each other).

It can be understood that the signal transmission circuit 26 may alternatively be disposed in the bracket 241 or in the compartment body 22, as long as the information read/write device 23 can interact with the information storage component 501 of the reagent container 50 placed in the target reagent position to obtain the corresponding reagent information.

In some implementations, the signal transmission circuit 26 corresponding to the non-target reagent position may be used as the electromagnetic field shielding circuit. For example, when the first reagent position 211a is the target reagent position, the first information read/write device 23a transmits the first electromagnetic field by means of the first signal transmission circuit 26a and the first antenna module 28a to the reagent container 50 in the first reagent position 211a, to perform signal interaction with the information storage component 501 of the reagent container 50 in the first reagent position 211a, so as to read reagent information stored in the information storage component 501 of the reagent container 50 in the first reagent position 211a. At this time, the second reagent position 211b is the non-target reagent position, and the second signal transmission circuit 26b is connected to the second antenna module 28b to generate the second counter electromagnetic field, so as to weaken the first electromagnetic field transmitted by the first information read/write device 23a via the first signal transmission circuit 26a and the first antenna module 28a to the second reagent position 211b, so that the information storage component 501 of the reagent container 50 in the second reagent position 211b cannot establish a communication connection with the first information read/write device 23a, thereby ensuring that the first information read/write device 23a can accurately read the reagent information in the information storage component 501 placed in the target reagent position. or, when the second reagent position 211b is the target reagent position, the second information read/write device 23b transmits the second electromagnetic field by means of the second signal transmission circuit 26b and the second antenna module 28b to the reagent container 50 in the second reagent position 211b, to perform signal interaction with the information storage component 501 of the reagent container 50 in the second reagent position 211b, so as to read reagent information stored in the information storage component 501 of the reagent container 50 in the second reagent position 211b. At this time, the first reagent position 211a is the non-target reagent position, and the first signal transmission circuit 26a is connected to the first antenna module 28a to generate the first counter electromagnetic field, so as to weaken the second electromagnetic field transmitted by the second information read/write device 23b via the second signal transmission circuit 26b and the second antenna module 28b to the first reagent position 211a, so that the information storage component 501 of the reagent container 50 in the first reagent position 211a cannot establish a communication connection with the second information read/write device 23b, thereby ensuring that the second information read/write device 23b can accurately read the reagent information in the information storage component 501 placed in the target reagent position.

In some other implementations, the electromagnetic field shielding circuit 25 and the signal transmission circuit 26 are independent of each other, and the electromagnetic field shielding circuit 25 include a third antenna module. The electromagnetic field shielding circuit 25 may generate a counter electromagnetic field by means of the third antenna module, to weaken the electromagnetic field transmitted to the non-target reagent position. For example, the signal transmission circuit 26 includes a first signal transmission circuit 26a and a second signal transmission circuit 26a independent of each other, and the electromagnetic field shielding circuit 25, the first signal transmission circuit 26a and the second signal transmission circuit 26a are independent of each other, as shown in FIG. 6.

It can be understand that, the electromagnetic field shielding circuit 25 and the signal transmission circuit 26 being independent of each other may be realized by the following ways: the electromagnetic field shielding circuit 25 include a first electromagnetic field shielding circuit 25a and a second electromagnetic field shielding circuit 25b independent of each other, and each of the first electromagnetic field shielding circuit 25a and the second electromagnetic field shielding circuit 25b includes a third antenna module, and the signal transmission circuit 26, first electromagnetic field shielding circuit 25a and the second electromagnetic field shielding circuit 25b are independent of each other; or the electromagnetic field shielding circuit 25 include a first electromagnetic field shielding circuit 25a and a second electromagnetic field shielding circuit 25b independent of each other, and each of the first electromagnetic field shielding circuit 25a and the second electromagnetic field shielding circuit 25b includes a third antenna module, the signal transmission circuit 26 includes a first signal transmission circuit 26a and a second signal transmission circuit 26a, wherein the first signal transmission circuit 26a, the second signal transmission circuit 26b, first electromagnetic field shielding circuit 25a and the second electromagnetic field shielding circuit 25b are independent of each other.

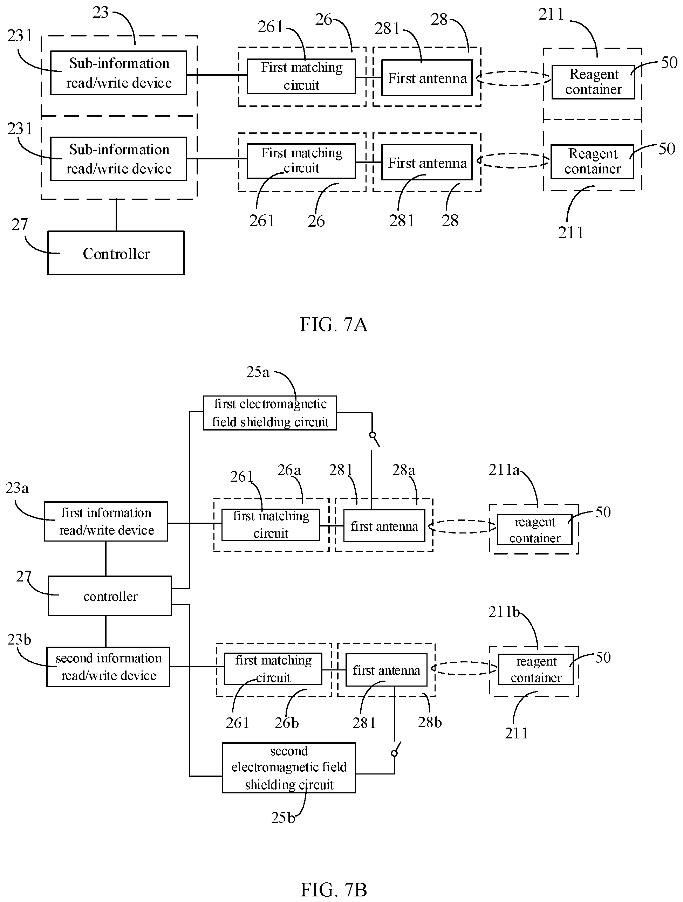

Referring to FIG. 7A, in some implementations, the information read/write device 23 includes several sub-information read/write devices 231 disposed corresponding to the reagent positions 211, and each sub-information read/write device 231 is electrically connected to the corresponding signal transmission circuit 26, so as to read or write information from or to the information storage component 501 in the corresponding reagent container 50 by means of the corresponding signal transmission circuit 26 and the antenna module 28. That is, as mentioned above in combination with FIG. 3, the first antenna module 28a, the first signal transmission circuit 26a, the first information read/write device 23a (i.e., a sub-information read/write device 231) and the first electromagnetic field shielding circuit 25a correspond to the first reagent position 211a, and the second antenna module 28b, the second signal transmission circuit 26b, the second information read/write device 23b (i.e., a sub-information read/write device 231) and the second electromagnetic field shielding circuit 25b correspond to the second reagent position 211b.

Specifically, in the implementations of FIG. 7A, each signal transmission circuit 26 disposed corresponding to the reagent position 211 includes a first matching circuit 261, and each antenna module 28 is provided with a first antenna 281 corresponding to the reagent position 211. A connection relationship between the sub-information read/write device 231, the first matching circuit 261, and the first antenna 281 corresponding to the reagent position 211 is that: each corresponding sub-information read/write device 231 is electrically connected to the first matching circuit 261, and the first matching circuit 261 is electrically connected to the first antenna 281, so as to read or write information from or to the information storage component 501 in the corresponding reagent position 211 by means of the first matching circuit 261 and the first antenna 281. Exemplarily, when the sub-information read/write device 231 is to read or write information from or to the information storage component 501 in the corresponding reagent position 211, the first antenna 281 is driven by the first matching circuit 261 to generate a corresponding electromagnetic field, and the interaction with the corresponding information storage component 501 is performed by using the electromagnetic field, so as to read or write information from or to the information storage component 501. A parameter, such as an electric field strength or an electric field direction, of the electric field emitted by the first antenna 281 can be adjusted by adjusting a circuit parameter of the first matching circuit 261 or a driving parameter of the sub-information read/write device 231.

In an example, as shown in FIG. 7B, each signal transmission circuit includes a first matching circuit 261, and each antenna module is provided with a first antenna 281, that is, the first signal transmission circuit 26a and the second signal transmission circuit 26b respectively include a first matching circuit 261, and the first antenna module 28a and the second antenna module 28b respectively include a first antenna 281. The first information read/write device 23a is connected to the first matching circuit 261 of the first signal transmission circuit 26a, and the first matching circuit 261 of the first signal transmission circuit 26a is capable of being connected to the first antenna 281of the first antenna module 28a, so as to perform signal interaction with the information storage component of the reagent container in the first reagent position 211a by means of the first matching circuit 261 of the first signal transmission circuit 26a and the first antenna 281 of the first antenna module 28a, to read or write information from or to the information storage component 501 of the reagent container 50 in the first reagent position 211a, and obtain reagent information stored in the information storage component 501 when reading information. The second information read/write device 23b is connected to the first matching circuit 261 of the second signal transmission circuit 26b, and the first matching circuit 261 of the second signal transmission circuit 26b is capable of being connected to the first antenna 281 of the second antenna module 28b, so as to perform signal interaction with the information storage component of the reagent container in the second reagent position 211b by means of the first matching circuit 261 of the second signal transmission circuit 26a and the first antenna 281 of the second antenna module 28a, to read or write information from or to the information storage component 501 of the reagent container 50 in the second reagent position 211b, and obtain reagent information stored in the information storage component 501 when reading information.

A parameter, such as an electric field strength or an electric field direction, of the electric field emitted by the first antenna 281 of the first antenna module 28a can be adjusted by adjusting a circuit parameter of the first matching circuit 261 of the first antenna module 28a or a driving parameter of the first information read/write device 23a. And A parameter, such as an electric field strength or an electric field direction, of the electric field emitted by the first antenna 281 of the second antenna module 28b can be adjusted by adjusting a circuit parameter of the first matching circuit 261 of the second antenna module 28b or a driving parameter of the second information read/write device 23b.

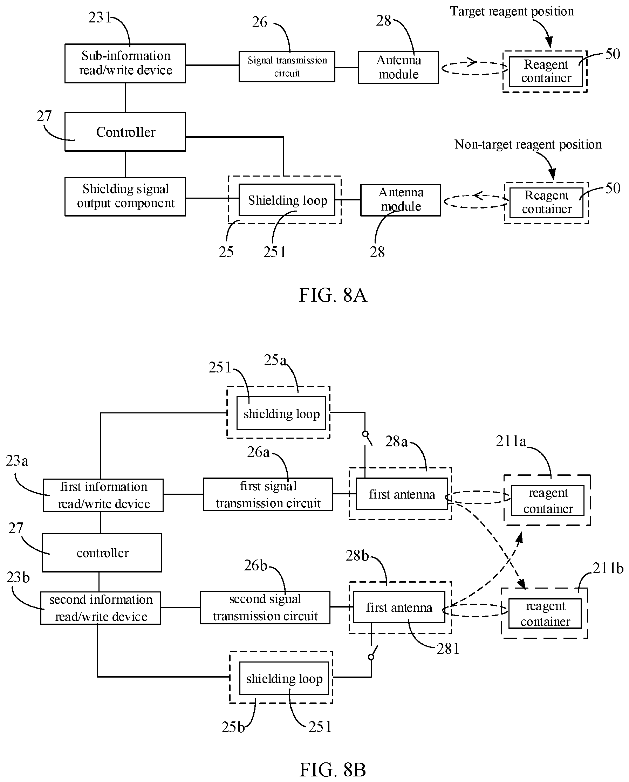

Referring to FIG. 8A, in some implementations, a method by which the controller 27 controls the electromagnetic field shielding circuit 25 to generate a counter electromagnetic field, so as to weaken an electromagnetic field emitted and transmitted by the signal transmission circuit 26 and the antenna module 28 corresponding to the target reagent position to another reagent position may be as follows: the electromagnetic field shielding circuit 25 is provided with a shielding loop 251; and when the information read/write device 23 transmits the emission electromagnetic field by means of the signal transmission circuit 26 and the antenna module 28 corresponding to the target reagent position, the controller 27 controls a current of the shielding loop 251 adjacent to the target reagent position to be greater than a preset current value, and a direction of the current to be opposite to that of a current of the signal transmission circuit 26 corresponding to the target reagent position.

A method of controlling the current of the shielding loop 251 may be as follows: the reagent supply device 20 is provided with a shielding signal output component connected to the controller 27. A shielding signal output terminal of the shielding signal output component is electrically connected to the shielding loop 251; and when the information read/write device 23 transmits the emission electromagnetic field by means of the signal transmission circuit 26 and the antenna module 28 corresponding to the target reagent position, the controller 27 controls the shielding signal output component to output a shielding signal, so that the current of the shielding loop 251 is greater than the preset current value, and the direction of the current is opposite to that of the current of the signal transmission circuit 26 corresponding to the target reagent position, so that the counter electromagnetic field generated by the shielding loop 251 in cooperation with the antenna module 28 can effectively weaken the electromagnetic field transmitted from the emission electromagnetic field to the another reagent position.

It can be understand that, the shielding signal output component may be a signal output element disposed independently of the sub-information read/write device 231, or may be the sub-information read/write device 231 corresponding to a non-target reagent position, which is temporarily used as a shielding signal output component when the sub-information read/write device 231 corresponding to the target reagent position transmits the emission electromagnetic field by means of the corresponding signal transmission circuit 26 and antenna module 28.

In this embodiment of the disclosure, the sub-information read/write device 231 corresponding to the reagent position 211 is taken as an example of the shielding signal output component for description, but the shielding signal output component is not necessarily the corresponding sub-information read/write device 231.

Exemplarily, the sub-information read/write device 231 corresponding to the reagent position 211 is further configured to output a shielding signal to adjust the current of the shielding loop 251, the shielding loop 251 corresponds to the reagent position 211, and a shielding signal output terminal of the sub-information read/write device 231 is electrically connected to the shielding loop 251; and when the information read/write device 23 transmits the emission electromagnetic field by means of the signal transmission circuit 26 and the antenna module 28 corresponding to the target reagent position, that is, when the sub-information read/write device 231 disposed corresponding to the target reagent position transmits the emission electromagnetic field by means of the signal transmission circuit 26 and the antenna module 28 corresponding to the target reagent position, the controller 27 controls the sub-information read/write device 231 adjacent to the target reagent position to output a shielding signal, so that the current of the shielding loop 251 adjacent to the target reagent position is greater than the preset current value, and the direction of the current is opposite to that of the current of the signal transmission circuit 26 corresponding to the target reagent position, thereby ensuring that the counter electromagnetic field generated by the shielding loop 251 can effectively weaken the emission electromagnetic field transmitted to the another reagent position adjacent to the target reagent position by the information read/write device 23 disposed corresponding target reagent position via the signal transmission circuit 26 and the antenna module 28. In this way, the emission electromagnetic field can activate the information storage component 501 placed in the target reagent position, to read the reagent information stored in the information storage component 501 in the target reagent position, and the emission electromagnetic field transmitted to the another reagent position adjacent to the target reagent position is not enough to interact with the information storage component 501 in the another reagent position, thereby ensuring that the information read/write device 23 can accurately read the reagent information in the corresponding information storage component 501 placed in the target reagent position.

In an example, referring to FIG. 8B, based on the implementations of FIG. 3, each electromagnetic field shielding circuit includes a shielding loop 251, that is, each of the first electromagnetic field shielding circuit 25a and the second electromagnetic field shielding circuit 25b includes a shielding loop 251. In this example, a method by which the controller 27 controls the electromagnetic field shielding circuit 25 to generate a counter electromagnetic field, so as to weaken an electromagnetic field emitted and transmitted by the signal transmission circuit 26 and the antenna module 28 corresponding to the target reagent position to another reagent position may be as follows:
when the first information read/write device 23a transmits the first electromagnetic field by means of the first signal transmission circuit 26a and the first antenna module 28a, the controller is further configured to control a current of the shielding loop 251 of the second electromagnetic field shielding circuit 25b to be greater than a preset current value, and a direction of said current to be opposite to that of a current of the first signal transmission circuit 26a; or
when the second information read/write device 23b transmits the second electromagnetic field by means of the second signal transmission circuit 26b and the second antenna module 28b, the controller is further configured to control a current of the shielding loop 251 of the first electromagnetic field shielding circuit 25a to be greater than a preset current value, and a direction of said current to be opposite to that of a current of the second signal transmission circuit 26b.

As shown in FIG. 8B, in some implementations, the shielding loop 251 of the first electromagnetic field shielding circuit 25a or the second electromagnetic field shielding circuit 25b may be controlled as follows: a shielding signal is outputted to the shielding loop 251 of the corresponding electromagnetic field shielding circuit by a shielding signal output component, to actively control the value of the current of the shielding loop 251 by adjusting a signal strength of the shielding signal.

For example, the information read/write device 23 corresponding to the non-target reagent position is used as the shielding signal output component, that is, when the first reagent position 211a is the target reagent position, the second information read/write device 23b may be used as the shielding signal output component; when the second reagent position 211b is the target reagent position, the first information read/write device 23a may be used as the shielding signal output component.

Specifically, each information read/write device is further configured to output a shielding signal to adjust the current of the corresponding shielding loop, and a shielding signal output terminal of each formation read/write device is electrically connected to the corresponding shielding loop. That is, the first information read/write device 23a is connected to the shielding loop 251 of the first electromagnetic field shielding circuit 25a, to adjust the current of the shielding loop 251 of the first electromagnetic field shielding circuit 25a, and the second information read/write device 23b is connected to the shielding loop 251 of the second electromagnetic field shielding circuit 25b, to adjust the current of the shielding loop 251 of the second electromagnetic field shielding circuit 25b.When the first information read/write device 23a transmits the first electromagnetic field by means of the first signal transmission circuit 26a and the first antenna module 28a, the controller 27 is further configured to control the second information read/write device 23b to output a shielding signal, so that the current of the shielding loop 251 of the second electromagnetic field shielding circuit 25b is greater than the preset current value, and the direction of said current is opposite to that of the current of the first signal transmission circuit 26a, so that the second counter electromagnetic field generated by the shielding loop 251 of the second electromagnetic field shielding circuit 25b in cooperation with the second antenna module 28b can effectively weaken the electromagnetic field transmitted from the first electromagnetic field to the second reagent position 211b. Or, when the second information read/write device 23b transmits the second electromagnetic field by means of the second signal transmission circuit 26b and the second antenna module 28b, the controller 27 is further configured to control the first information read/write device 23a to output a shielding signal, so that the current of the shielding loop 251 of the first electromagnetic field shielding circuit 25a is greater than the preset current value, and the direction of said current is opposite to that of the current of the second signal transmission circuit 26b, so that the first counter electromagnetic field generated by the shielding loop 251 of the first electromagnetic field shielding circuit 25a in cooperation with the first antenna module 28a can effectively weaken the electromagnetic field transmitted from the second electromagnetic field to the first reagent position 211a.

In some implementations, the first antenna 281 of the antenna module 28 corresponding to the reagent position 211 is further configured to transmit the counter electromagnetic field, and when the information read/write device 23 transmits the emission electromagnetic field by means of the signal transmission circuit 26 and the antenna module 28 corresponding to the target reagent position, the controller 27 controls the first antenna 281 of the antenna module 28 adjacent to the target reagent position to emit the counter electromagnetic field.

Specifically, when the information read/write device 23 transmits the emission electromagnetic field by means of the signal transmission circuit 26 and the antenna module 28 corresponding to the target reagent position, the controller 27 controls the corresponding shielding signal output component to output the shielding signal, that is, controls the sub-information read/write device 231 adjacent to the target reagent position to output the shielding signal. At this time, the sub-information read/write device 231 generates and emits the counter electromagnetic field by means of the corresponding shielding loop 251 and first antenna 281, so as to weaken the electromagnetic field diffused from the emission electromagnetic field to the another reagent position adjacent to the target reagent position.

Preferably, the shielding signal that is output by the sub-information read/write device 231 makes the current of the shielding loop 251 adjacent to the target reagent position greater than the preset current value, and the direction of the current opposite to that of the current of the signal transmission circuit 26 corresponding to the target reagent position.

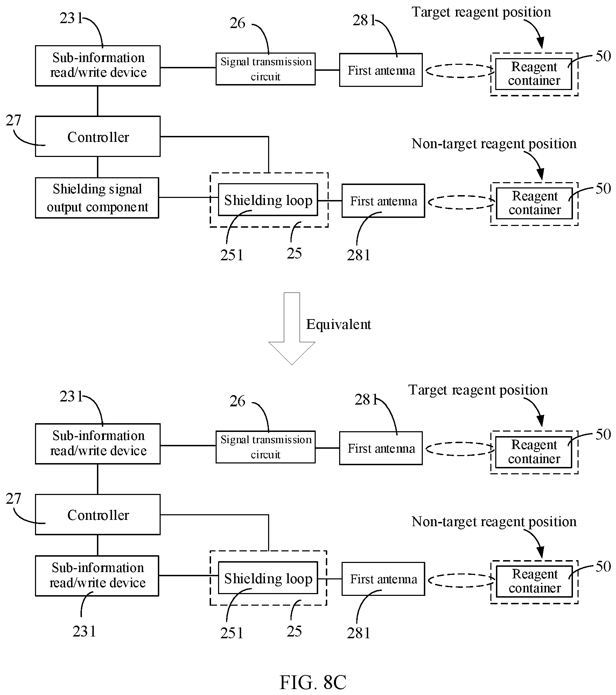

Referring to FIG. 8C, exemplarily, each reagent position 211 is correspondingly provided with a first antenna 281, and each first antenna 281 has at least two working modes. To be specific, the two working modes include a first working mode for cooperating with the signal transmission circuit 261 to output the emission electromagnetic field, and a second working mode for cooperating with the signal shielding loop 251 to output the counter electromagnetic field, wherein switching between the first working mode and the second working mode is determined according to the corresponding target reagent position.

Specifically, when the reagent information of the reagent container 50 placed in the target reagent position is to be obtained, the controller 27 controls the information read/write device 23 to transmit the emission electromagnetic field by means of the signal transmission circuit 26 and the first antenna 281 corresponding to the target reagent position, so as to interact with the information storage component 501 of the reagent container 50 in the target reagent position to obtain the corresponding reagent information.

At this time, the controller 27 controls the sub-information read/write device 231 corresponding to the reagent position 211 adjacent to the target reagent position to cooperate with the corresponding shielding loop 251 and the first antenna 281 to generate the counter electromagnetic field, so as to weaken the emission electromagnetic field transmitted from the first antenna 281 corresponding to the target reagent position to the adjacent reagent position, so that the emission electromagnetic field transmitted from the first antenna 281 disposed corresponding to the target reagent position is not enough to activate the information storage component 501 in the another reagent position and establish a communication connection with the information storage component 501 in the another reagent position for interaction, thereby ensuring that the sub-information read/write device 231 disposed corresponding to the target reagent position can interact only with the information storage component 501 in the target reagent position to obtain the reagent information corresponding to the reagent container 50 placed in the corresponding target reagent position.

In other words, when the sub-information read/write device 231 disposed corresponding to the target reagent position performs signal interaction with the information storage component 501 of the reagent container 50 placed in the target reagent position, the sub-information read/write device 231 corresponding to the non-target reagent position adjacent to the target reagent position may temporarily serve as a shielding signal output component, and outputs the counter electromagnetic field by means of the corresponding shielding loop 251 and the first antenna 281 corresponding to the non-target reagent position adjacent to the target reagent position.

As shown in FIG. 8B, each first antenna is further configured to transmit the counter electromagnetic field, that is, the first antenna 281 of the first antenna module 28a and the first antenna 281 of the second antenna module 28b are further configured to transmit the corresponding counter electromagnetic field. The first electromagnetic field shielding circuit 25a is capable of being connected to the first antenna 281 of the first antenna module 28a to generate the first counter electromagnetic field, and the second electromagnetic field shielding circuit 25b is capable of being connected to the first antenna 281 of the second antenna module 28b to generate the second counter electromagnetic field. When the first information read/write device 23a transmits the first electromagnetic field by means of the first signal transmission circuit 26a and the first antenna module 28a, the controller 27 is further configured to control the second electromagnetic field shielding circuit 25b to be connected to the first antenna 281 of the second antenna module 28b to generate the second counter electromagnetic field; or when the second information read/write device 23b transmits the second electromagnetic field by means of the second signal transmission circuit 26b and the second antenna module 28b, the controller 27 is further configured to control the first electromagnetic field shielding circuit 25a to be connected to the first antenna 281 of the first antenna module 28a to generate the first counter electromagnetic field.

For example, when the first reagent position 211a is the target reagent position, and the first information read/write device 23a transmits the first electromagnetic field by means of the first signal transmission circuit 26a and the first antenna module 28a, the controller 27 is further configured to control the second information read/write device 23b to output a shielding signal, so that the second information read/write device 23b generates and emits the second counter electromagnetic field by means of the shielding loop 251 of the second electromagnetic field shielding circuit 25b and the first antenna 281 of the second antenna module 28b, so as to weaken the electromagnetic field diffused from the first electromagnetic field to the non-target reagent position (for example the second reagent position 211b). Or, when the second reagent position 211b is the target reagent position, and the second information read/write device 23b transmits the second electromagnetic field by means of the second signal transmission circuit 26b and the second antenna module 28b, the controller 27 is further configured to control the first information read/write device 23a to output a shielding signal, so that the first information read/write device 23a generates and emits the first counter electromagnetic field by means of the shielding loop 251 of the first electromagnetic field shielding circuit 25a and the first antenna 281 of the first antenna module 28a, so as to weaken the electromagnetic field diffused from the second electromagnetic field to the non-target reagent position (for example the first reagent position 211a).

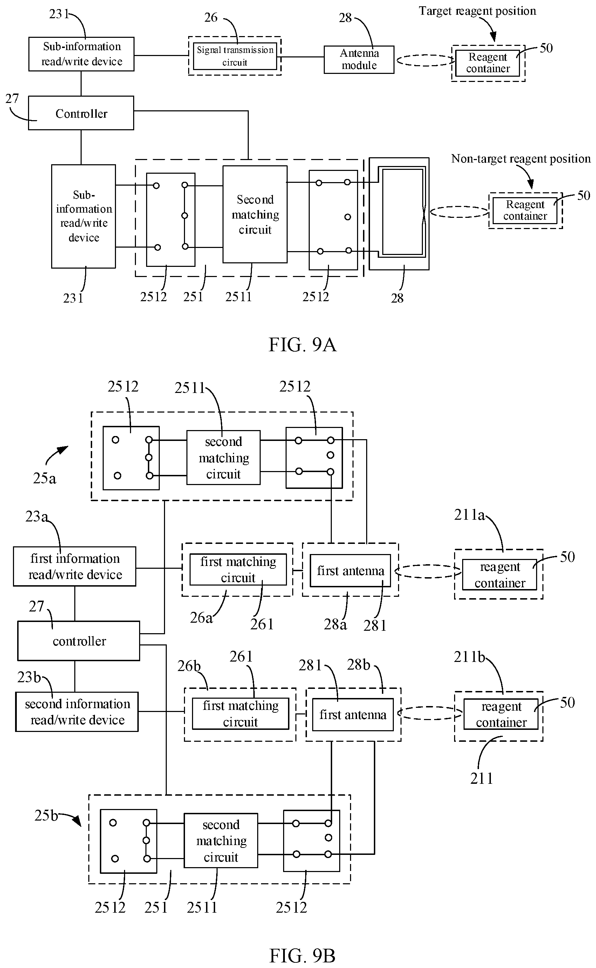

Referring to FIG. 9A, in some implementations, the shielding loop 251 includes a second matching circuit 2511 and a switch module 2512, wherein an impedance of the second matching circuit 2511 is less than that of the first matching circuit 261.

A method by which the controller 27 controls the current of the shielding loop 251 adjacent to the target reagent position to be greater than the preset current value, and the direction of the current to be opposite to that of the current of the signal transmission circuit 26 corresponding to the target reagent position may be as follows: when the information read/write device 23 transmits the emission electromagnetic field by means of the signal transmission circuit 26 and the antenna module 28 corresponding to the target reagent position, the controller 27 controls a switch module 252 adjacent to the target reagent position to electrically connect the second matching circuit 251 to the antenna module 28, and the second matching circuit 2511 and the antenna module 28 form a closed loop, so that an impedance of the shielding loop 251 adj acent to the target reagent position is less than that of the signal transmission circuit 26 corresponding to the target reagent position.

As shown in FIG. 9A, in this implementation, since the second matching circuit 2511 and the antenna module 28 form a closed loop, when the closed loop receives the emission electromagnetic field emitted and transmitted to the another reagent position by the information read/write device 23 corresponding to the target reagent position via the signal transmission circuit 26 and the antenna module 28, since the antenna module 28 adjacent to the target reagent position is in the emission electromagnetic field generated by the signal transmission circuit 26, and the impedance of the shielding loop 251 adjacent to the target reagent position is less than that of the signal transmission circuit 26 corresponding to the target reagent position, it can be known from Lenz's law that the antenna module 28 in the emission electromagnetic field and adjacent to the target reagent position can generate an induced current with a current value greater than the preset current value and a current direction opposite to that of the current of the signal transmission circuit 26 corresponding to the target reagent position, and the induced current generates a counter electromagnetic field with a direction opposite to that of the emission electromagnetic field, so as to weaken the emission electromagnetic field emitted and transmitted to the another reagent position by the information read/write device 23 corresponding to the target reagent position via the signal transmission circuit 26 and the antenna module 28, so that the emission electromagnetic field transmitted to the another reagent position is not enough to activate the information storage component 501 in the another reagent position, thereby ensuring that the information read/write device 23 can interact only with the information storage component 501 in the target reagent position by means of the signal transmission circuit 26 and the antenna module 28, so as to obtain the reagent information corresponding to the reagent container 50 placed in the corresponding target reagent position.

In an example, referring to FIG. 9B, based on the implementations shown in FIG. 7B, each electromagnetic field shielding circuit includes a shielding loop 251, and each shielding loop 251 includes a second matching circuit 2511 and a switch module 2512, wherein an impedance of the second matching circuit 2511 is less than that of the first matching circuit 261. That is, each shielding loop 251 of the first electromagnetic field shielding circuit 25a and the second electromagnetic field shielding circuit 25b includes a second matching circuit 2511 and a switch module 2512, wherein an impedance of the second matching circuit 2511 of the first electromagnetic field shielding circuit 25a is less than that of the first matching circuit 261 of the first signal transmission circuit 26a, and an impedance of the second matching circuit 2511 of the second electromagnetic field shielding circuit 25b is less than that of the first matching circuit 261 of the second signal transmission circuit 26b.

When the first reagent position 211a is the target reagent position, and the first information read/write device 23a transmits the first electromagnetic field to the first reagent position 211a by means of the first signal transmission circuit 26a and the first antenna module 28a, the controller 27 is further configured to control the switch module 2512 of the shielding loop 251 of the second electromagnetic field shielding circuit 25b to connect the second matching circuit 2511 of the shielding loop 251 of the second electromagnetic field shielding circuit 25b to the second antenna module 28b, so that the second matching circuit 2511 of the shielding loop 251 of the second electromagnetic field shielding circuit 25b and the second antenna module 28b form a closed loop, wherein an impedance of the shielding loop 251 of the second electromagnetic field shielding circuit 25b is less than that of the first signal transmission circuit 26a.

Since the second matching circuit 2511 of the second electromagnetic field shielding circuit 25b and the second antenna module 28b form a closed loop, when the closed loop receives the first electromagnetic field emitted and transmitted to the another reagent position (for example the second reagent position 211b) by the first information read/write device 23a corresponding to the target reagent position via the first signal transmission circuit 26a and the first antenna module 28a, since the second antenna module 28b adjacent to the target reagent position is in the first electromagnetic field generated by the first signal transmission circuit 26a, and the impedance of the shielding loop 251 of the second electromagnetic field shielding circuit 25b adjacent to the target reagent position is less than that of the first signal transmission circuit 26a corresponding to the target reagent position, it can be known from Lenz's law that the second antenna module 28b in the first electromagnetic field and adjacent to the target reagent position can generate an induced current with a current value greater than the preset current value and a current direction opposite to that of the current of the first signal transmission circuit 26a corresponding to the target reagent position, and the induced current generates the second counter electromagnetic field with a direction opposite to that of the first electromagnetic field, so as to weaken the first electromagnetic field emitted and transmitted to the another reagent position (for example the second reagent position 211b) by the first information read/write device 23a corresponding to the target reagent position via the first signal transmission circuit 26a and the first antenna module 28a, so that the first electromagnetic field transmitted to the another reagent position is not enough to activate the information storage component 501 in the another reagent position, thereby ensuring that the first information read/write device 23a can interact only with the information storage component 501 in the target reagent position by means of the first signal transmission circuit 26a and the first antenna module 28a, so as to obtain the reagent information corresponding to the reagent container 50 placed in the corresponding target reagent position.

Alternatively, when the second reagent position 211b is the target reagent position, and the second information read/write device 23b transmits the second electromagnetic field to the second reagent position 211b by means of the second signal transmission circuit 26b and the second antenna module 28b, the controller 27 is further configured to control the switch module 2512 of the shielding loop 251 of the first electromagnetic field shielding circuit 25a to connect the second matching circuit 2511 of the shielding loop 251 of the first electromagnetic field shielding circuit 25a to the first antenna module 28a, so that the second matching circuit 2511 of the shielding loop 251 of the first electromagnetic field shielding circuit 25a and the first antenna module 28a form a closed loop, wherein an impedance of the shielding loop 251 of the first electromagnetic field shielding circuit 25a is less than that of the second signal transmission circuit 26b.

Since the second matching circuit 2511 of the first electromagnetic field shielding circuit 25a and the first antenna module 28a form a closed loop, when the closed loop receives the second electromagnetic field emitted and transmitted to the another reagent position (for example the first reagent position 211a) by the second information read/write device 23b corresponding to the target reagent position via the second signal transmission circuit 26b and the second antenna module 28b, since the first antenna module 28a adjacent to the target reagent position is in the second electromagnetic field generated by the second signal transmission circuit 26b, and the impedance of the shielding loop 251 of the first electromagnetic field shielding circuit 25a adjacent to the target reagent position is less than that of the second signal transmission circuit 26b corresponding to the target reagent position, it can be known from Lenz's law that the first antenna module 28a in the second electromagnetic field and adjacent to the target reagent position can generate an induced current with a current value greater than the preset current value and a current direction opposite to that of the current of the second signal transmission circuit 26b corresponding to the target reagent position, and the induced current generates the first counter electromagnetic field with a direction opposite to that of the second electromagnetic field, so as to weaken the second electromagnetic field emitted and transmitted to the another reagent position (for example the first reagent position 211a) by the second information read/write device 23b corresponding to the target reagent position via the second signal transmission circuit 26b and the second antenna module 28b, so that the second electromagnetic field transmitted to the another reagent position is not enough to activate the information storage component 501 in the another reagent position, thereby ensuring that the second information read/write device 23b can interact only with the information storage component 501 in the target reagent position by means of the second signal transmission circuit 26b and the second antenna module 28b, so as to obtain the reagent information corresponding to the reagent container 50 placed in the corresponding target reagent position.

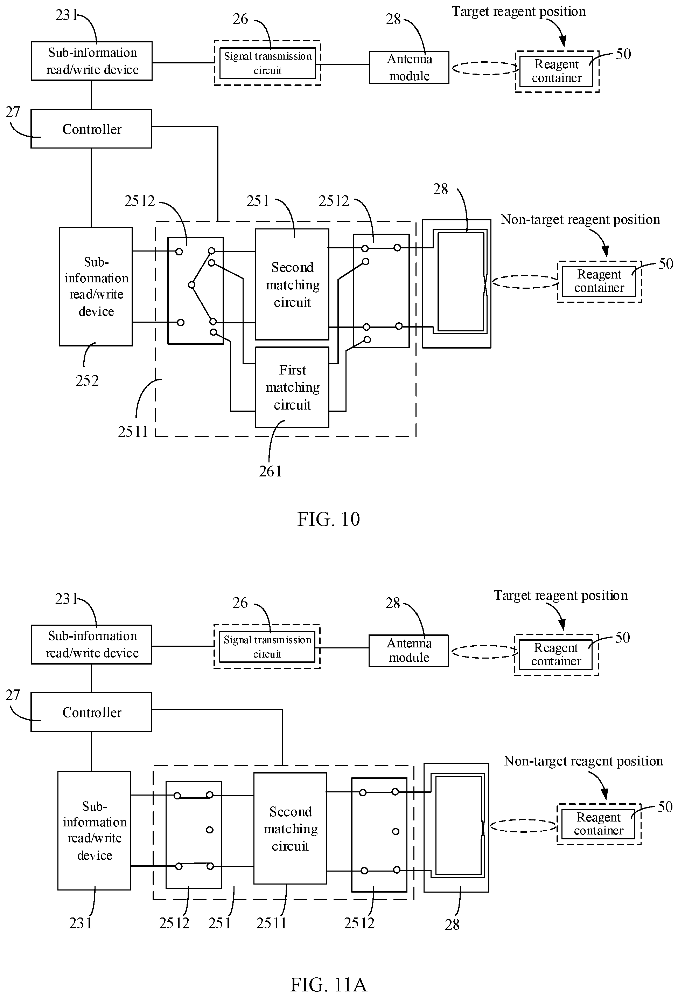

As shown in FIG. 10, this implementation differs from the implementation corresponding to FIG. 9A in that the shielding loop 251 further includes the first matching circuit 261.

To be specific, in this implementation, the shielding loop 251 includes the second matching circuit 2511, the first matching circuit 261, and the switch module 2512, where the impedance of the second matching circuit 2511 is less than that of the first matching circuit 261. When the information read/write device 23 transmits the emission electromagnetic field by means of the signal transmission circuit 26 and the antenna module 28 corresponding to the target reagent position, the controller 27 controls the switch module 252 adjacent to the target reagent position to electrically connect the second matching circuit 251 to the antenna module 28, and the second matching circuit 2511 and the antenna module 28 form a closed loop, so that an impedance of the shielding loop 251 adjacent to the target reagent position is less than that of the signal transmission circuit 26 corresponding to the target reagent position.

Referring to FIG. 11A, in some implementations, the shielding loop 251 includes a second matching circuit 2511 and a switch module 2512, and each sub-information read/write device 231 is further configured to output a shielding signal.

A method by which the controller 27 controls the current of the shielding loop 251 adjacent to the target reagent position to be greater than the preset current value, and the direction of the current to be opposite to that of the current of the signal transmission circuit 26 corresponding to the target reagent position may be as follows: when the information read/write device 23 transmits the emission electromagnetic field by means of the signal transmission circuit 26 and the antenna module 28 corresponding to the target reagent position, the controller 27 controls the switch module 2512 adjacent to the target reagent position to electrically connect the second matching circuit 2511 to the antenna module 28, and controls the switch module 2512 to electrically connect the second matching circuit 2511 to the sub-information read/write device 231; and the controller 27 further controls the sub-information read/write device 231 to output a shielding signal.

As shown in FIG. 11A, in this implementation, when the information read/write device 23 transmits the emission electromagnetic field by means of the signal transmission circuit 26 and the antenna module 28 corresponding to the target reagent position, that is, when the sub-information read/write device 231 disposed corresponding to the target reagent position transmits the emission electromagnetic field by means of the signal transmission circuit 26 and the antenna module 28 corresponding to the target reagent position, the controller 27 controls the switch module 2512 adjacent to the target reagent position to electrically connect the second matching circuit 2511 to the antenna module 28, and controls the switch module 2512 to electrically connect the second matching circuit 2511 to the sub-information read/write device 231; and the controller 27 further controls the sub-information read/write device 231 to output a shielding signal, so that the current of the shielding loop 251 adjacent to the target reagent position is greater than the preset current value, and the direction of the current is opposite to that of the current of the signal transmission circuit 26 corresponding to the target reagent position, such that the counter electromagnetic field generated by the second matching circuit 2511 of the shielding loop 251 and the antenna module 28 is enough to weaken the emission electromagnetic field.

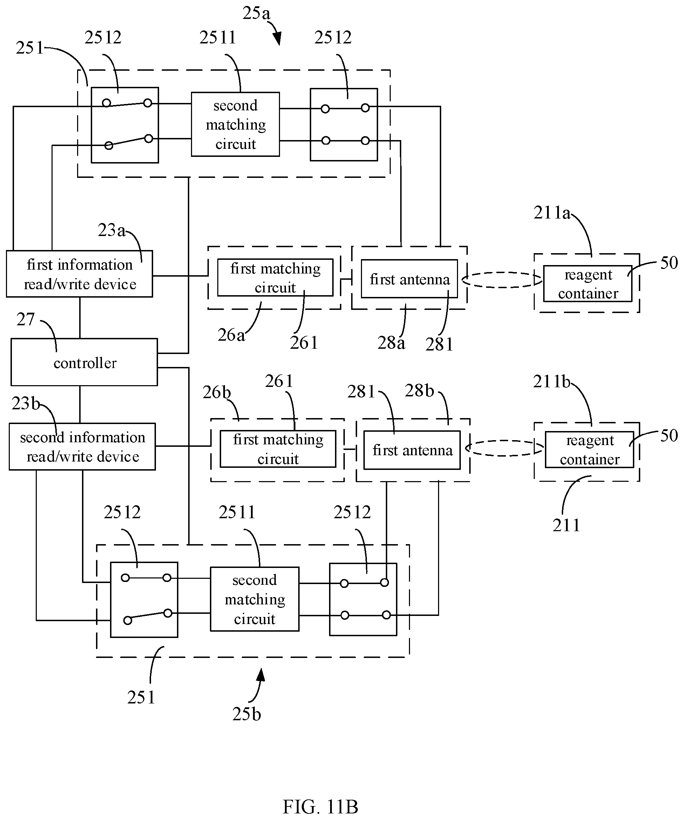

In an example, as shown in FIG. 11B, based on the implementations shown in FIG. 7B, each electromagnetic field shielding circuit includes a shielding loop 251, and each shielding loop 251 includes a second matching circuit 2511 and a switch module 2512, and each of the first information read/write device 23a and the second information read/write device 23b is further configured to output a shielding signal.

Specifically, when the first reagent position 211a is the target reagent position, and the first information read/write device 23a transmits the first electromagnetic field by means of the first signal transmission circuit 26a and the first antenna module 28a, the controller 27 is further configured to control the switch module 2512 of the shielding loop 251 of the second electromagnetic field shielding circuit 25b to connect the second matching circuit 2511 of the shielding loop 251 of the second electromagnetic field shielding circuit 25b to the second antenna module 28b, control the switch module 2512 of the shielding loop 251 of the second electromagnetic field shielding circuit 25b to connect the second matching circuit 2511 of the shielding loop 521 of the second electromagnetic field shielding circuit 25b to the second information read/write device 23b, and control the second information read/write device 23b to output a shielding signal to the second electromagnetic field shielding circuit 25b, so that for example the current of the shielding loop 251 of the second electromagnetic field shielding circuit 25b is greater than the preset current value, and the direction of said current is opposite to that of the current of the first signal transmission circuit 26a. In this way, the second counter electromagnetic field generated by the shielding loop 251 of the second electromagnetic field shielding circuit 25b and the second antenna module 28b is sufficient to weaken the first electromagnetic field emitted and transmitted to the second reagent position 211b by the first information read/write device 23a via the first signal transmission circuit 26a and the first antenna module 28a.

Alternatively, when the second reagent position 211b is the target reagent position, and the second information read/write device 23b transmits the second electromagnetic field by means of the second signal transmission circuit 26b and the second antenna module 28b, the controller 27 is further configured to control the switch module 2512 of the shielding loop 251 of the first electromagnetic field shielding circuit 25a to connect the second matching circuit 2511 of the shielding loop 251 of the first electromagnetic field shielding circuit 25a to the first antenna module 28a, control the switch module 2512 of the shielding loop 251 of the first electromagnetic field shielding circuit 25a to connect the second matching circuit 2511 of the shielding loop 251 of the first electromagnetic field shielding circuit 25a to the first information read/write device 23a, and control the first information read/write device 23a to output a shielding signal to the first electromagnetic field shielding circuit 25a, so that for example the current of the shielding loop 251 of the first electromagnetic field shielding circuit 25a is greater than the preset current value, and the direction of said current is opposite to that of the current of the second signal transmission circuit 26b. In this way, the first counter electromagnetic field generated by the shielding loop 251 of the first electromagnetic field shielding circuit 25a and the second antenna module 28a is sufficient to weaken the second electromagnetic field emitted and transmitted to the first reagent position 211a by the second information read/write device 23b via the second signal transmission circuit 26b and the second antenna module 28b.

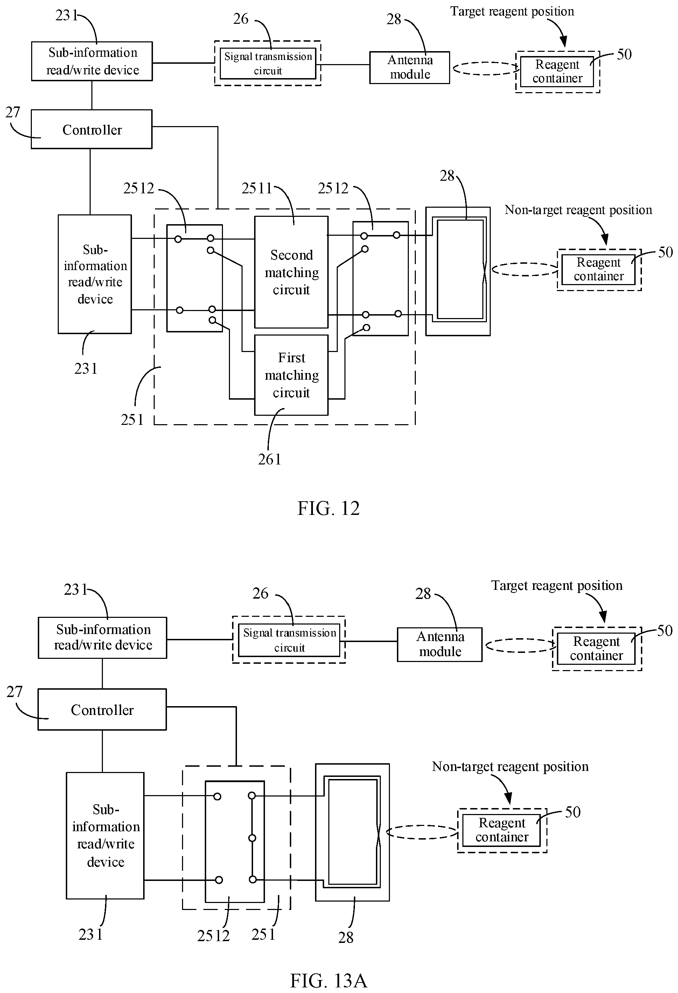

As shown in FIG. 12, this implementation differs from the implementation provided in FIG. 11A in that the shielding loop 251 further includes the first matching circuit 261, and each sub-information read/write device 231 is further configured to output a shielding signal.

To be specific, in this implementation, the shielding loop 251 includes the second matching circuit 2511, the first matching circuit 261, and the switch module 2512, wherein the impedance of the second matching circuit 2511 is less than that of the first matching circuit 261. When the information read/write device 23 transmits the emission electromagnetic field by means of the signal transmission circuit 26 and the antenna module 28 corresponding to the target reagent position, the controller 27 controls the switch module 2512 adjacent to the target reagent position to electrically connect the second matching circuit 2511 to the antenna module 28, and controls the switch module 2512 to electrically connect the second matching circuit 2511 to the sub-information read/write device 231; and the controller 27 further controls the sub-information read/write device 231 to output a shielding signal.

Referring to FIG. 13A, in some implementations, the shielding loop includes a switch module 2512.

A method by which the controller 27 controls the current of the shielding loop 251 adjacent to the target reagent position to be greater than the preset current value, and the direction of the current to be opposite to that of the current of the signal transmission circuit 26 corresponding to the target reagent position may be as follows: when the information read/write device 23 transmits the emission electromagnetic field by means of the signal transmission circuit 26 and the antenna module 28 corresponding to the target reagent position, the controller 27 controls the switch module 2512 adjacent to the target reagent position to be closed, so that the switch module 2512 and the antenna module 28 form a closed loop, so that an impedance of the shielding loop 251 adjacent to the target reagent position is less than that of the signal transmission circuit 26 corresponding to the target reagent position.

As shown in FIG. 13A, in this implementation, since the switch module 2512 and the antenna module 28 form a closed loop, when the closed loop receives the emission electromagnetic field emitted and transmitted to the another reagent position by the information read/write device 23 corresponding to the target reagent position via the signal transmission circuit 26 and the antenna module 28, since the antenna module 28 adjacent to the target reagent position is in the electromagnetic field generated by the signal transmission circuit 26, and the impedance of the shielding loop 251 adjacent to the target reagent position is less than that of the signal transmission circuit 26 corresponding to the target reagent position, it can be known from Lenz's law that the antenna module 28 in the emission electromagnetic field and adjacent to the target reagent position can generate an induced current with a current value greater than the preset current value and a current direction opposite to that of the current of the signal transmission circuit 26 corresponding to the target reagent position, and the induced current generates a counter electromagnetic field with a direction opposite to that of the emission electromagnetic field, so as to weaken the emission electromagnetic field emitted and transmitted to the another reagent position by the information read/write device 23 corresponding to the target reagent position by means of the signal transmission circuit 26 and the antenna module 28, so that the emission electromagnetic field transmitted to the another reagent position is not enough to activate the information storage component 501 in the another reagent position, thereby ensuring that the information read/write device 23 can interact only with the information storage component 501 in the target reagent position by means of the signal transmission circuit 26 and the antenna module 28, so as to obtain the reagent information corresponding to the reagent container 50 placed in the corresponding target reagent position.

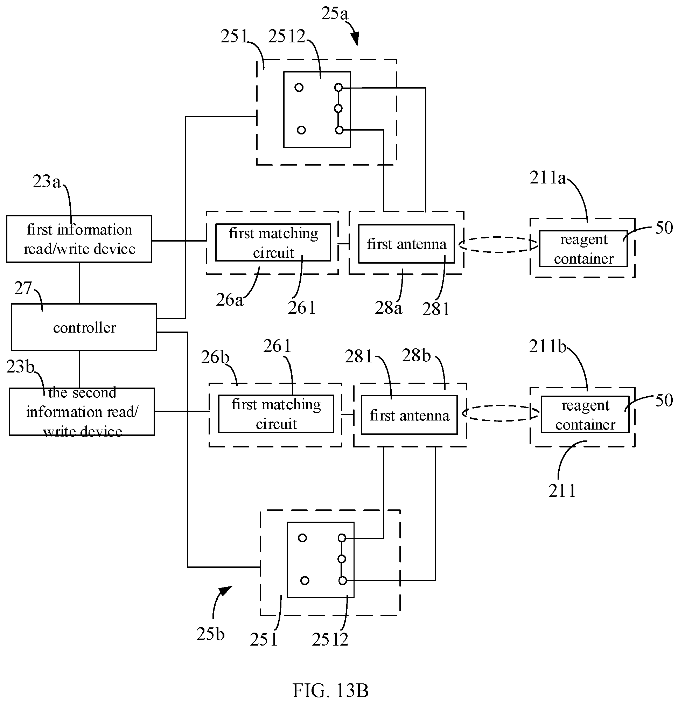

In a example, as shown in FIG. 13B, based on the implementations shown in FIG. 7B, each electromagnetic field shielding circuit includes a shielding loop 251, and each shielding loop 251 includes a switch module 2512. That is, each shielding loop 251 of the first electromagnetic field shielding circuit 25a and the second electromagnetic field shielding circuit 25b includes a switch module 2512.

When the first reagent position 211a is the target reagent position, and the first information read/write device 23a transmits the first electromagnetic field to the first reagent position 211a by means of the first signal transmission circuit 26a and the first antenna module 28a, the controller 27 is further configured to control the switch module 2512 of the shielding loop 251 of the second electromagnetic field shielding circuit 25a to be closed, so that the switch module 2512 of the shielding loop 251 of the second electromagnetic field shielding circuit 25b and the second antenna module 28b form a closed loop, wherein an impedance of the shielding loop 251 of the second electromagnetic field shielding circuit 25a is less than that of the first signal transmission circuit 26a.

Since the switch module 2512 of the shielding loop 251 of the second electromagnetic field shielding circuit 25b and the second antenna module 28b form a closed loop, when the closed loop receives the first electromagnetic field emitted and transmitted to the another reagent position (for example the second reagent position 211b) by the first information read/write device 23a corresponding to the target reagent position via the first signal transmission circuit 26a and the first antenna module 28a, since the second antenna module 28b adjacent to the target reagent position is in the first electromagnetic field generated by the first signal transmission circuit 26a, and the impedance of the shielding loop 251 of the second electromagnetic field shielding circuit 25b adjacent to the target reagent position is less than that of the first signal transmission circuit 26a corresponding to the target reagent position, it can be known from Lenz's law that the second antenna module 28b in the first electromagnetic field and adjacent to the target reagent position can generate an induced current with a current value greater than the preset current value and a current direction opposite to that of the current of the first signal transmission circuit 26a corresponding to the target reagent position, and the induced current generates the second counter electromagnetic field with a direction opposite to that of the first electromagnetic field, so as to weaken the first electromagnetic field emitted and transmitted to the another reagent position (for example the second reagent position 211b) by the first information read/write device 23a corresponding to the target reagent position via the first signal transmission circuit 26a and the first antenna module 28a, so that the first electromagnetic field transmitted to the another reagent position is not enough to activate the information storage component 501 in the another reagent position, thereby ensuring that the first information read/write device 23a can interact only with the information storage component 501 in the target reagent position by means of the first signal transmission circuit 26a and the first antenna module 28a, so as to obtain the reagent information corresponding to the reagent container 50 placed in the corresponding target reagent position.

Alternatively, when the second reagent position 211b is the target reagent position, and the second information read/write device 23b transmits the second electromagnetic field to the second reagent position 211b by means of the second signal transmission circuit 26b and the second antenna module 28b, the controller 27 is further configured to control the switch module 2512 of the shielding loop 251 of the first electromagnetic field shielding circuit 25a to be closed, so that the switch module 2512 of the shielding loop 251 of the first electromagnetic field shielding circuit 25a and the first antenna module 28a form a closed loop, wherein an impedance of the shielding loop 251 of the first electromagnetic field shielding circuit 25a is less than that of the second signal transmission circuit 26b.

Since the switch module 2512 of the shielding loop 251 of the first electromagnetic field shielding circuit 25a and the first antenna module 28a form a closed loop, when the closed loop receives the second electromagnetic field emitted and transmitted to the another reagent position (for example the first reagent position 211a) by the second information read/write device 23b corresponding to the target reagent position via the second signal transmission circuit 26b and the second antenna module 28b, since the first antenna module 28a adjacent to the target reagent position is in the second electromagnetic field generated by the second signal transmission circuit 26b, and the impedance of the shielding loop 251 of the first electromagnetic field shielding circuit 25a adjacent to the target reagent position is less than that of the second signal transmission circuit 26b corresponding to the target reagent position, it can be known from Lenz's law that the first antenna module 28a in the second electromagnetic field and adjacent to the target reagent position can generate an induced current with a current value greater than the preset current value and a current direction opposite to that of the current of the second signal transmission circuit 26b corresponding to the target reagent position, and the induced current generates the first counter electromagnetic field with a direction opposite to that of the second electromagnetic field, so as to weaken the second electromagnetic field emitted and transmitted to the another reagent position (for example the first reagent position 211a) by the second information read/write device 23b corresponding to the target reagent position via the second signal transmission circuit 26b and the second antenna module 28b, so that the second electromagnetic field transmitted to the another reagent position is not enough to activate the information storage component 501 in the another reagent position, thereby ensuring that the second information read/write device 23b can interact only with the information storage component 501 in the target reagent position by means of the second signal transmission circuit 26b and the second antenna module 28b, so as to obtain the reagent information corresponding to the reagent container 50 placed in the corresponding target reagent position.

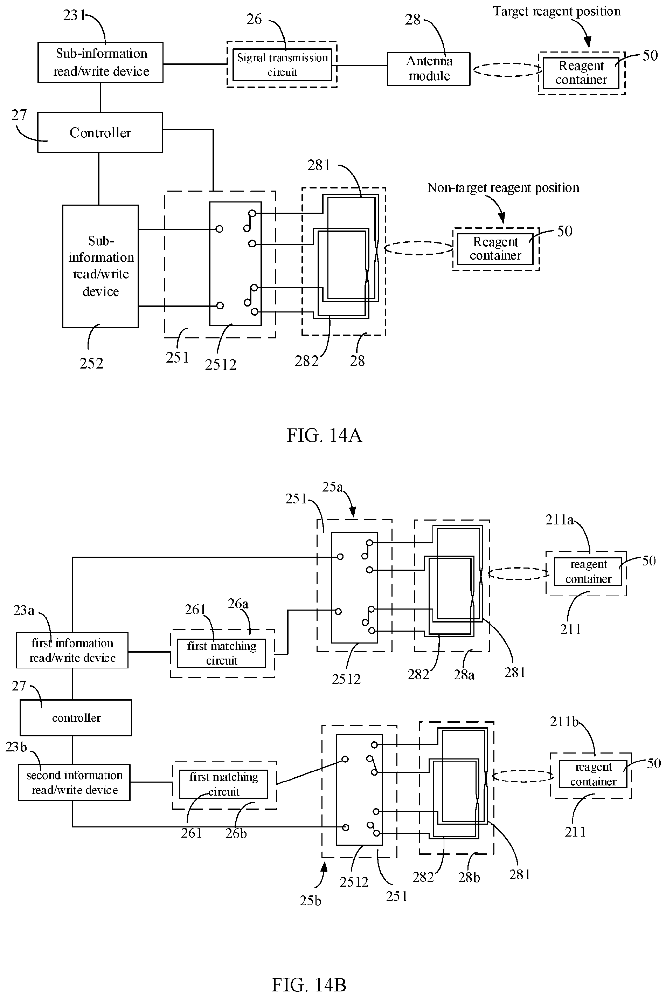

Referring to FIG. 14A, in some implementations, the antenna module 28 includes a first antenna 281 and a second antenna 282, and the shielding loop 251 includes a switch module 2512, wherein an impedance value of the first antenna 281 is less than that of the second antenna 282, and each sub-information read/write device 231 is further configured to output a shielding signal.

A method by which the controller 27 controls the current of the shielding loop 251 adjacent to the target reagent position to be greater than the preset current value, and the direction of the current to be opposite to that of the current of the signal transmission circuit 26 corresponding to the target reagent position may be as follows: when the information read/write device 23 transmits the emission electromagnetic field by means of the signal transmission circuit 26 and the antenna module 28 corresponding to the target reagent position, the controller 27 controls the switch module 2512 adjacent to the target reagent position to connect the first antenna 281 and the sub-information read/write device 231, and controls the sub-information read/write device 231 to output a shielding signal, so that the current of the shielding loop 251 adjacent to the target reagent position is greater than the preset current value, and the direction of the current is opposite to that of the current of the signal transmission circuit 26 corresponding to the target reagent position, such that the counter electromagnetic field generated by the shielding loop 251 and the first antenna 281 is enough to weaken the emission electromagnetic field.

In a example, as shown in FIG. 14B, based on the implementations shown in FIG. 7B, each antenna module is further provided with a second antenna 282, and each second antenna 282 is configured to transmit the corresponding counter electromagnetic field. Each electromagnetic field shielding circuit includes a shielding loop 251, and each shielding loop 251 includes a switch module 2512. The first electromagnetic field shielding circuit 25a is capable of being connected to the second antenna 282 of the first antenna module 28a to generate the first counter electromagnetic field, and the second electromagnetic field shielding circuit 25b is capable of being connected to the second antenna 282of the second antenna module 28b to generate the second counter electromagnetic field.

When the first information read/write device 23a transmits the first electromagnetic field by means of the first signal transmission circuit 26a and the first antenna 281 of the first antenna module 28a, the controller 27 is further configured to control the second electromagnetic field shielding circuit 25b to be connected to the second antenna 282 of the second antenna module 28b to generate the second counter electromagnetic field; or when the second information read/write device 23b transmits the second electromagnetic field by means of the second signal transmission circuit 26b and the first antenna 281 of the second antenna module 28b, the controller 27 is further configured to control the first electromagnetic field shielding circuit 25a to be connected to the second antenna 282 of the first antenna module 28a to generate the first counter electromagnetic field.

Further, when the first information read/write device 23a transmits the first electromagnetic field by means of the first signal transmission circuit 26a and the first antenna 281 of the first antenna module 28a, the controller 27 is further configured to control the second information read/write device 23b to output a shielding signal, and control the switch module 2512 to connect the shielding loop 251 of the second electromagnetic field shielding circuit 25b to the second antenna 282 of the second antenna module 28b, to drive the second electromagnetic field shielding circuit 25b and the second antenna 282 of the second antenna module 28b to generate the second counter electromagnetic field. Similarly, when the second information read/write device 23b transmits the second electromagnetic field by means of the second signal transmission circuit 26b and the first antenna 281 of the second antenna module 28b, the controller 27 is further configured to control the first information read/write device 23a to output a shielding signal, and control the switch module 2512 to connect the shielding loop 251 of the first electromagnetic field shielding circuit 25a to the second antenna 282 of the first antenna module 28a, to drive the first electromagnetic field shielding circuit 25a and the second antenna 282 of the first antenna module 28a to generate the first counter electromagnetic field.

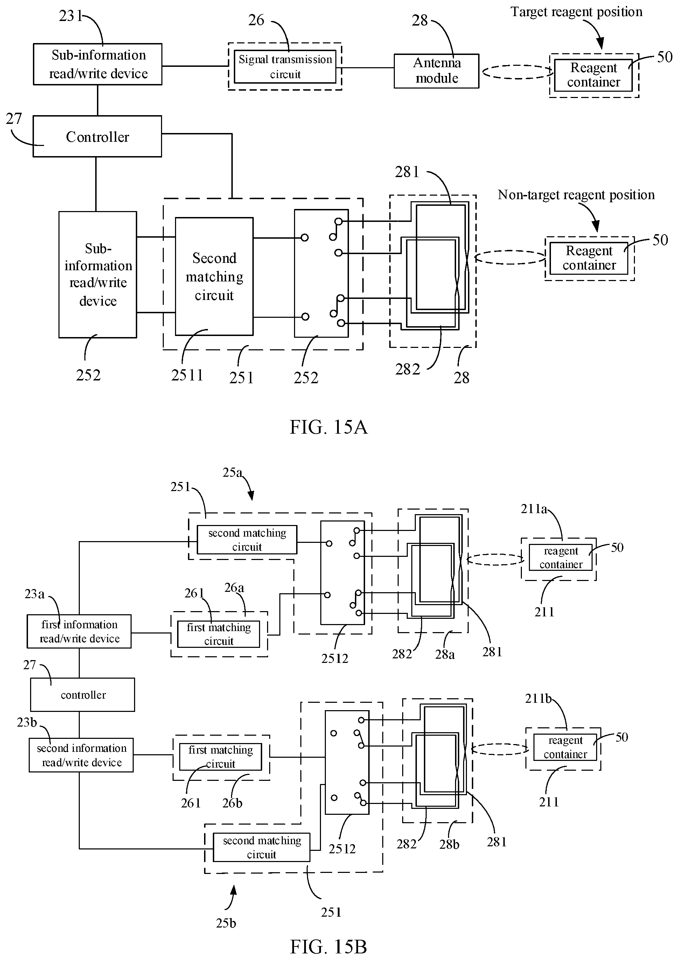

Referring to FIG. 15A, in some implementations, the antenna module 28 includes a first antenna 281 and a second antenna 282, and the shielding loop 251 includes a switch module 2512 and a second matching circuit 2511, wherein an impedance value of the first antenna 281 is less than that of the second antenna 282. And each sub-information read/write device 231 is further configured to output a shielding signal

A method by which the controller 27 controls the current of the shielding loop 251 adjacent to the target reagent position to be greater than the preset current value, and the direction of the current to be opposite to that of the current of the signal transmission circuit 26 corresponding to the target reagent position may be as follows: the controller 27 controls the switch module 2512 adjacent to the target reagent position to connect the first antenna 281 and the second matching circuit 2511, and controls the switch module 2512 to connect the second matching circuit 2511 and the sub-information read/write device 231; and at the same time, controls the sub-information read/write device 231 to output a shielding signal, so that the current of the shielding loop 251 adjacent to the target reagent position is greater than the preset current value, and the direction of the current is opposite to that of the current of the signal transmission circuit 26 corresponding to the target reagent position, such that the counter electromagnetic field generated by the shielding loop 251 and the first antenna 281 is enough to weaken the emission electromagnetic field.

In an example, as shown in FIG. 15B, based on the implementations shown in FIG. 7B, each antenna module is further provided with a second antenna 282, and each second antenna 282 is configured to transmit the corresponding counter electromagnetic field. Each electromagnetic field shielding circuit includes a shielding loop 251, and each shielding loop 251 includes a second matching circuit 2511 and a switch module 2512. The first electromagnetic field shielding circuit 25a is capable of being connected to the second antenna 282 of the first antenna module 28a to generate the first counter electromagnetic field, and the second electromagnetic field shielding circuit 25b is capable of being connected to the second antenna 282of the second antenna module 28b to generate the second counter electromagnetic field.

When the first information read/write device 23a transmits the first electromagnetic field by means of the first matching circuit 261 of the first signal transmission circuit 26a and the first antenna 281 of the first antenna module 28a, the controller 27 is further configured to control the second matching circuit 2511 of the second electromagnetic field shielding circuit 25b to be connected to the second antenna 282 of the second antenna module 28b to generate the second counter electromagnetic field; or when the second information read/write device 23b transmits the second electromagnetic field by means of the first matching circuit 261 of the second signal transmission circuit 26b and the first antenna 281 of the second antenna module 28b, the controller 27 is further configured to control the second matching circuit 2511 of the first electromagnetic field shielding circuit 25a to be connected to the second antenna 282 of the first antenna module 28a to generate the first counter electromagnetic field.

Further, when the first information read/write device 23a transmits the first electromagnetic field by means of the first matching circuit 261 of the first signal transmission circuit 26a and the first antenna 281 of the first antenna module 28a, the controller 27 is further configured to control the second information read/write device 23b to output a shielding signal, and control the switch module 2512 to connect the second matching circuit 2511 of the shielding loop 251 of the second electromagnetic field shielding circuit 25b to the second antenna 282 of the second antenna module 28b, to drive the first matching circuit 2511 of the second electromagnetic field shielding circuit 25b and the second antenna 282 of the second antenna module 28b to generate the second counter electromagnetic field. Similarly, when the second information read/write device 23b transmits the second electromagnetic field by means of the first matching circuit 261 of the second signal transmission circuit 26b and the first antenna 281 of the second antenna module 28b, the controller 27 is further configured to control the first information read/write device 23a to output a shielding signal, and control the switch module 2512 to connect the second matching circuit 2511 of the shielding loop 251 of the first electromagnetic field shielding circuit 25a to the second antenna 282 of the first antenna module 28a, to drive the second matching circuit 2511 of the first electromagnetic field shielding circuit 25a and the second antenna 282 of the first antenna module 28a to generate the first counter electromagnetic field.

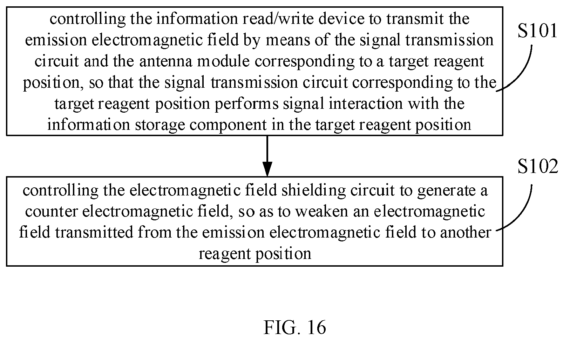

Referring to FIG. 16, an embodiment of the disclosure provides a reagent information reading method. The reagent information reading method is applied to the sample analyzer.

In this embodiment, the reagent information reading method being applied to a sample analyzer is taken as an example for description, the reagent information reading method includes steps S101 and S102.

Step S101: controlling the information read/write device to transmit the emission electromagnetic field by means of the signal transmission circuit and the antenna module corresponding to a target reagent position, so that the signal transmission circuit corresponding to the target reagent position performs signal interaction with the information storage component in the target reagent position.

When the information read/write device transmits the emission electromagnetic field by means of the signal transmission circuit and the antenna module corresponding to the target reagent position, or when a reagent testing instruction is received, the information read/write device is controlled to transmit the emission electromagnetic field by means of the signal transmission circuit and the antenna module corresponding to the target reagent position, so that the signal transmission circuit corresponding to the target reagent position performs signal interaction with an information storage component in the target reagent position, to obtain the reagent information stored in the information storage component. The reagent testing instruction may be generated by triggering a corresponding information input device disposed on the reagent supply device or the sample analyzer, and the information input device includes, but is not limited to, a button, a keyboard, a touch display screen. Alternatively, the reagent testing instruction may be periodically generated and sent to the controller by the reagent supply device or the sample analyzer, which is not limited herein.

Exemplarily, when reagent information corresponding to a reagent stored in a corresponding reagent container is to be obtained, the information input device is used to deliver a corresponding reagent testing instruction to the reagent supply device. When the reagent supply device receives the reagent testing instruction, the controller controls the information read/write device to generate the emission electromagnetic field by means of the signal transmission circuit corresponding to the target reagent position, so that the signal transmission circuit corresponding to the target reagent position performs signal interaction with the information storage component in the target reagent position, so that the corresponding information read/write device obtains the reagent information stored in the information storage component.

Step S102: controlling the electromagnetic field shielding circuit to generate a counter electromagnetic field, so as to weaken an electromagnetic field transmitted from the emission electromagnetic field to another reagent position.

When receiving the reagent testing instruction, the controller further controls the electromagnetic field shielding circuit to generate a counter electromagnetic field, so as to weaken the electromagnetic field emitted and transmitted to another reagent position by the signal transmission circuit corresponding to the target reagent position in cooperation with the antenna module.

Exemplarily, when receiving the reagent testing instruction, the controller further controls the electromagnetic field shielding circuit to generate a counter electromagnetic field by means of the corresponding antenna module, so as to weaken the electromagnetic field emitted and transmitted to the another reagent position by the signal transmission circuit corresponding to the target reagent position in cooperation with the antenna module, so that the electromagnetic field emitted by the signal transmission circuit is not enough to activate the information storage component in the another reagent position, thereby ensuring that the signal transmission circuit corresponding to the target reagent can interact only with the information storage component in the target reagent position, and the corresponding information read/write device obtains the reagent information stored in the information storage component.

Exemplarily, the reagent information reading method is applied to the sample analyzer shown in FIG.3. When the first reagent position 211a is the target reagent position, step S101 includes: controlling the first information read/write device to transmit the first electromagnetic field by means of the first signal transmission circuit and the first antenna module to the information storage component of the reagent container in the first reagent position, so that the first information read/write device performs signal interaction with the information storage component of the reagent container in the first reagent position; and step S 102 includes: controlling the second electromagnetic field shielding circuit to be connected to the second antenna module to generate the second counter electromagnetic field, so as to weaken an electromagnetic field transmitted from the first electromagnetic field to the second reagent position. When the second reagent position 211b is the target reagent position, step S101 includes: controlling the second information read/write device to transmit the second electromagnetic field by means of the second signal transmission circuit and the second antenna module to the information storage component of the reagent container in the second reagent position, so that the second information read/write device performs signal interaction with the information storage component of the reagent container in the second reagent position; and step S102 includes: controlling the first electromagnetic field shielding circuit to be connected to the first antenna module to generate the first counter electromagnetic field, so as to weaken an electromagnetic field transmitted from the second electromagnetic field to the first reagent position.

In some implementations, the execution order between step S101 and step S102 may be interchanged.

In some implementations, controlling the electromagnetic field shielding circuit to generate a counter electromagnetic field, so as to weaken an electromagnetic field transmitted from the emission electromagnetic field to another reagent position specifically includes: adjusting a current of the electromagnetic field shielding circuit, so that a direction of the current of the electromagnetic field shielding circuit is opposite to that of a current of the signal transmission circuit corresponding to the target reagent position.

Exemplarily, controlling the second electromagnetic field shielding circuit to generate the second counter electromagnetic field, so as to weaken an electromagnetic field transmitted from the first electromagnetic field to the second reagent position comprises: adjusting a current of the second electromagnetic field shielding circuit, so that a direction of the current of the second electromagnetic field shielding circuit is opposite to that of a current of the first signal transmission circuit; or controlling the first electromagnetic field shielding circuit to generate the first counter electromagnetic field, so as to weaken an electromagnetic field transmitted from the second electromagnetic field to the first reagent position comprises: adjusting a current of the first electromagnetic field shielding circuit, so that a direction of the current of the first electromagnetic field shielding circuit is opposite to that of a current of the second signal transmission circuit.

In some implementations, controlling the electromagnetic field shielding circuit to generate a counter electromagnetic field, so as to weaken an electromagnetic field transmitted from the emission electromagnetic field to another reagent position specifically includes: adjusting a current of the electromagnetic field shielding circuit, so that a direction of the current of the electromagnetic field shielding circuit is opposite to that of a current of the signal transmission circuit corresponding to the target reagent position, and the current of the electromagnetic field shielding circuit is greater than or equal to that of the signal transmission circuit corresponding to the target reagent position.

Exemplarily, controlling the second electromagnetic field shielding circuit to generate the second counter electromagnetic field, so as to weaken an electromagnetic field transmitted from the first electromagnetic field to the second reagent position comprises: adjusting a current of the second electromagnetic field shielding circuit, so that a direction of the current of the second electromagnetic field shielding circuit is opposite to that of a current of the first signal transmission circuit, and the current of the second electromagnetic field shielding circuit is greater than or equal to that of the first signal transmission circuit; or controlling the first electromagnetic field shielding circuit to generate the first counter electromagnetic field, so as to weaken an electromagnetic field transmitted from the second electromagnetic field to the first reagent position comprises: adjusting a current of the first electromagnetic field shielding circuit, so that a direction of the current of the first electromagnetic field shielding circuit is opposite to that of a current of the second signal transmission circuit, and the current of the first electromagnetic field shielding circuit is greater than or equal to that of the second signal transmission circuit.

It should be understood that the terms used in the description of the disclosure are only intended to describe specific embodiments, but not to limit the disclosure. As used in the description and the appended claims of the disclosure, unless the context clearly indicates otherwise, the singular forms "a", "an" and "the" are intended to include the plural forms.

It should also be understood that the term "and/or" used in the description and the appended claims of the disclosure refers to one of the items listed correlatively or any combination and all possible combinations of the items, and includes these combinations. It should be noted that the term "comprise", "include", or any other variant thereof herein is intended to encompass a non-exclusive inclusion, such that a process, method, article, or system that includes a series of elements not only includes those elements, but also includes other elements not explicitly listed, or elements that are inherent to such a process, method, article, or system. In the absence of more restrictions, an element defined by "including a ..." does not exclude another same element in a process, method, article, or system that includes the element.

The serial numbers of the above embodiments of the disclosure are merely for description, and do not represent the superiority or inferiority of the embodiments. The above descriptions are merely the specific implementations of the disclosure, but the scope of protection of the disclosure is not limited thereto.

Therefore, the scope of protection of the disclosure shall be subject to the scope of protection of the claims.

## Claims

1. A sample analyzer (100), comprising:
a sample supply device (10) configured to supply a blood sample to be tested;
a reagent supply device (20) configured to supply a reagent for reacting with the blood sample to be tested;
a reaction container (30) configured to receive the blood sample to be tested that is supplied by the sample supply device (10) and the reagent that is supplied by the reagent supply device (20), so that the blood sample to be tested and the reagent react to form a mixed sample solution; and
a testing device (40) comprising a testing region (401) made of a light-transmitting material and a light source (402) corresponding to the testing region (401), the light source (402) being configured to irradiate the mixed sample solution flowing through the testing region (401), so as to measure content of a specific protein in the mixed sample solution;
wherein the reagent supply device (20) comprises:
a reagent placement compartment (21) provided with at least two reagent positions (211), wherein the at least two reagent positions (211) are used for respectively placing a reagent container (50) containing a reagent, and the reagent container (50) is provided with an information storage component (501) storing reagent information;
an antenna module (28);
a signal transmission circuit (26) capable of being connected to the antenna module (28) to transmit an emission electromagnetic field;
an information read/write device (23) connected to the signal transmission circuit (26) and configured to perform signal interaction with the information storage component (501) of the reagent container (50) in each reagent position (211), so as to read or write information from or to the information storage component (501) of the reagent container (50); and
a controller (27) configured to:
control the information read/write device (23) to transmit the emission electromagnetic field by means of the signal transmission circuit (26) and the antenna module (28), so that the information read/write device (23) performs signal interaction with the information storage component (501) of the reagent container (50) in a first reagent position (211a) of the at least two reagent positions (211);
**characterized in that** the reagent supply device (20) further comprises:
an electromagnetic field shielding circuit (25) configured to generate a counter electromagnetic field; and
**in that** the controller (27) is further configured to:
control the electromagnetic field shielding circuit (25) to generate the counter electromagnetic field, so as to weaken an electromagnetic field transmitted from the emission electromagnetic field to a second reagent position (211b) of the at least two reagent positions (211).

2. The sample analyzer (100) of claim 1, **characterized in that**,
the antenna module (28) comprises a first antenna module (28a) corresponding to the first reagent position (211a) and a second antenna module (28b) corresponding to the second reagent position (211b), the signal transmission circuit (26) comprises a first signal transmission circuit (26a) corresponding to the first reagent position (211a) and a second signal transmission circuit (26b) corresponding to the second reagent position (211b), the information read/write device (23) comprises a first information read/write device (23a) corresponding to the first reagent position (211a) and a second information read/write device (23b) corresponding to the second reagent position (211b), and the electromagnetic field shielding circuit (25) comprises a first electromagnetic field shielding circuit (25a) corresponding to the first reagent position (211a) and a second electromagnetic field shielding circuit (25b) corresponding to the second reagent position (211b);
the first information read/write device (23a) is capable of being connected to the first antenna module (28a) via the first signal transmission circuit (26a), so as to transmit a first electromagnetic field to the information storage component of the reagent container in the first reagent position (211a) and perform signal interaction with the information storage component of the reagent container in the first reagent position (211a), and the first electromagnetic field shielding circuit (25a) is capable of being connected to the first antenna module (28a) to generate a first counter electromagnetic field;
the second information read/write device (23b) is capable of being connected to the second antenna module (28b) via the second signal transmission circuit (26b), so as to transmit a second electromagnetic field to the information storage component of the reagent container in the second reagent position (211b) and perform signal interaction with the information storage component of the reagent container in the second reagent position (211b), and the second electromagnetic field shielding circuit (25b) is capable of being connected to the second antenna module (28b) to generate a second counter electromagnetic field; and
the controller (27) is further configured to:
control the first information read/write device (23a) to transmit the first electromagnetic field by means of the first signal transmission circuit (26a) and the first antenna module (28a) to the information storage component of the reagent container in the first reagent position (211a), so that the first information read/write device (23a) performs signal interaction with the information storage component of the reagent container in the first reagent position (211a); and control the second electromagnetic field shielding circuit (25b) to be connected to the second antenna module (28b) to generate the second counter electromagnetic field, so as to weaken an electromagnetic field transmitted from the first electromagnetic field to the second reagent position (211b);
or
control the second information read/write device (23b) to transmit the second electromagnetic field by means of the second signal transmission circuit (26b) and the second antenna module (28b) to the information storage component of the reagent container in the second reagent position (211b), so that the second information read/write device (23b) performs signal interaction with the information storage component of the reagent container in the second reagent position (211b); and control the first electromagnetic field shielding circuit (25a) to be connected to the first antenna module (28a) to generate the first counter electromagnetic field, so as to weaken an electromagnetic field transmitted from the second electromagnetic field to the first reagent position (211a).

3. The sample analyzer (100) of claim 2, **characterized in that** a field direction of the second counter electromagnetic field generated by the second electromagnetic field shielding circuit (25b) is opposite to that of the first electromagnetic field transmitted by the first information read/write device (23a) via the first signal transmission circuit (26a) and the first antenna module (28a); or
a field direction of the first counter electromagnetic field generated by the first electromagnetic field shielding circuit (25a) is opposite to that of the second electromagnetic field transmitted by the second information read/write device (23b) via the second signal transmission circuit (26b) and the second antenna module (28b).

4. The sample analyzer (100) of claim 3, **characterized in that** a field strength of the second counter electromagnetic field generated by the second electromagnetic field shielding circuit (25b) is greater than or equal to a field strength of the first electromagnetic field transmitted by the first information read/write device (23a) via the first signal transmission circuit (26a) and the first antenna module (28a); or
a field strength of the first counter electromagnetic field generated by the first electromagnetic field shielding circuit (25a) is greater than or equal to a field strength of the second electromagnetic field transmitted by the second information read/write device (23b) via the second signal transmission circuit (26b) and the second antenna module (28b).

5. The sample analyzer (100) of any one of claims 2 to 4, **characterized in that** the first signal transmission circuit (26a) and the second signal transmission circuit (26b) are independent of each other; and/or, the first electromagnetic field shielding circuit (25a) and the second electromagnetic field shielding circuit (25b) are independent of each other; and/or, the first information read/write device (23a) and the second information read/write device (23b) are independent of each other.

6. The sample analyzer (100) of any one of claims 2 to 5, **characterized in that**
each signal transmission circuit (26) comprises a first matching circuit (261), and each antenna module (28) is provided with a first antenna (281);
wherein the first information read/write device (23a) is connected to the first matching circuit (261) of the first signal transmission circuit (26a), and the first matching circuit (261) of the first signal transmission circuit (26a) is capable of being connected to the first antenna (281) of the first antenna module (28a), so as to perform signal interaction with the information storage component of the reagent container in the first reagent position (211a) by means of the first matching circuit (261) of the first signal transmission circuit (26a) and the first antenna (281) of the first antenna module (28a); and
wherein the second information read/write device (23b) is connected to the first matching circuit (261) of the second signal transmission circuit (26b), and the first matching circuit (261) of the second signal transmission circuit (26b) is capable of being connected to the first antenna (281) of the second antenna module (28b), so as to perform signal interaction with the information storage component of the reagent container in the second reagent position by means of the first matching circuit (261) of the second signal transmission circuit (26b) and the first antenna (281) of the second antenna module (28b).

7. The sample analyzer (100) of claim 6, **characterized in that** each electromagnetic field shielding circuit (25) comprises a shielding loop (251); and
when the first information read/write device (23a) transmits the first electromagnetic field by means of the first signal transmission circuit (26a) and the first antenna module (28a), the controller (27) is further configured to control a current of the shielding loop (251) of the second electromagnetic field shielding circuit (25b) to be greater than a preset current value, and a direction of said current to be opposite to that of a current of the first signal transmission circuit (26a); or
when the second information read/write device (23b) transmits the second electromagnetic field by means of the second signal transmission circuit (26b) and the second antenna module (28b), the controller (27) is further configured to control a current of the shielding loop (251) of the first electromagnetic field shielding circuit (25a) to be greater than a preset current value, and a direction of said current to be opposite to that of a current of the second signal transmission circuit (26b).

8. The sample analyzer (100) of claim 7, **characterized in that** each information read/write device (23) is further configured to output a shielding signal to adjust the current of the corresponding shielding loop (251), and a shielding signal output terminal of each information read/write device (23) is electrically connected to the corresponding shielding loop (251); and
when the first information read/write device (23a) transmits the first electromagnetic field by means of the first signal transmission circuit (26a) and the first antenna module (28a), the controller (27) is further configured to control the second information read/write device (23b) to output a shielding signal, so that the current of the shielding loop (251) of the second electromagnetic field shielding circuit (25b) is greater than the preset current value, and the direction of said current is opposite to that of the current of the first signal transmission circuit (26a);
or when the second information read/write device (23b) transmits the second electromagnetic field by means of the second signal transmission circuit (26b) and the second antenna module (28b), the controller (27) is further configured to control the first information read/write device (23a) to output a shielding signal, so that the current of the shielding loop (251) of the first electromagnetic field shielding circuit (25a) is greater than the preset current value, and the direction of said current is opposite to that of the current of the second signal transmission circuit (26b).

9. The sample analyzer (100) of claim 7, **characterized in that** each shielding loop (251) comprises a second matching circuit (2511) and a switch module (2512), wherein an impedance of the second matching circuit (2511) is less than that of the first matching circuit; and
when the first information read/write device (23a) transmits the first electromagnetic field by means of the first signal transmission circuit (26a) and the first antenna module (28a), the controller (27) is further configured to control the switch module (2512) of the shielding loop (251) of the second electromagnetic field shielding circuit (25b) to connect the second matching circuit (2511) of the shielding loop (251) of the second electromagnetic field shielding circuit (25b) to the second antenna module (28b), so that the second matching circuit (2511) of the shielding loop (251) of the second electromagnetic field shielding circuit (25b) and the second antenna module (28b) form a closed loop, wherein an impedance of the shielding loop (251) of the second electromagnetic field shielding circuit (25b) is less than that of the first signal transmission circuit (26a); or
when the second information read/write device (23b) transmits the second electromagnetic field by means of the second signal transmission circuit (26b) and the second antenna module (28b), the controller (27) is further configured to control the switch module (2512) of the shielding loop (251) of the first electromagnetic field shielding circuit (25a) to connect the second matching circuit (2511) of the shielding loop (251) of the first electromagnetic field shielding circuit (25a) to the first antenna module (28a), so that the second matching circuit (2511) of the shielding loop (251) of the first electromagnetic field shielding circuit (25a) and the first antenna module (28a) form a closed loop, wherein an impedance of the shielding loop (251) of the first electromagnetic field shielding circuit (25a) is less than that of the second signal transmission circuit (26b).

10. The sample analyzer (100) of claim 7, **characterized in that** each shielding loop (251) comprises a switch module (2512); and
when the first information read/write device (23a) transmits the first electromagnetic field by means of the first signal transmission circuit (26a) and the first antenna module (28a), the controller (27) is further configured to control the switch module (2512) of the shielding loop (251) of the second electromagnetic field shielding circuit (25b) to be closed, so that the switch module (2512) of the shielding loop (251) of the second electromagnetic field shielding circuit (25b) and the second antenna module (28b) form a closed loop, wherein an impedance of the shielding loop (251) of the second electromagnetic field shielding circuit (25b) is less than that of the first signal transmission circuit (26a); or
when the second information read/write device (23b) transmits the second electromagnetic field by means of the second signal transmission circuit (26b) and the second antenna module (28b), the controller (27) is further configured to control the switch module (2512) of the shielding loop (251) of the first electromagnetic field shielding circuit (25a) to be closed, so that the switch module (2512) of the shielding loop (251) of the first electromagnetic field shielding circuit (25a) and the first antenna module (28a) form a closed loop, wherein an impedance of the shielding loop (251) of the first electromagnetic field shielding circuit (25a) is less than that of the second signal transmission circuit (26b).

11. The sample analyzer (100) of claim 7, **characterized in that** each shielding loop (251) comprises a second matching circuit (2511) and a switch module (2512); and
when the first information read/write device (23a) transmits the first electromagnetic field by means of the first signal transmission circuit (26a) and the first antenna module (28a), the controller (27) is further configured to control the switch module (2512) of the shielding loop (251) of the second electromagnetic field shielding circuit (25b) to connect the second matching circuit (2511) of the shielding loop (251) of the second electromagnetic field shielding circuit (25b) to the second antenna module (28b), control the switch module (2512) of the shielding loop (251) of the second electromagnetic field shielding circuit (25b) to connect the second matching circuit (2511) of the shielding loop (251) of the second electromagnetic field shielding circuit (25b) to the second information read/write device (23b), and control the second information read/write device (23b) to output a shielding signal to the second electromagnetic field shielding circuit (25b); or
when the second information read/write device (23b) transmits the second electromagnetic field by means of the second signal transmission circuit (26b) and the second antenna module (28b), the controller (27) is further configured to control the switch module (2512) of the shielding loop (251) of the first electromagnetic field shielding circuit (25a) to connect the second matching circuit (2511) of the shielding loop (251) of the first electromagnetic field shielding circuit (25a) to the first antenna module (28a), control the switch module (2512) of the shielding loop (251) of the first electromagnetic field shielding circuit (25a) to connect the second matching circuit (2511) of the shielding loop (251) of the first electromagnetic field shielding circuit (25a) to the first information read/write device (23a), and control the first information read/write device (23a) to output a shielding signal to the first electromagnetic field shielding circuit (25a).

12. The sample analyzer (100) of any one of claims 6 to 11, **characterized in that** each first antenna (281) is further configured to transmit the counter electromagnetic field, wherein the first electromagnetic field shielding circuit (25a) is capable of being connected to the first antenna (281) of the first antenna module (28a) to generate the first counter electromagnetic field, and the second electromagnetic field shielding circuit (25b) is capable of being connected to the first antenna (281) of the second antenna module (28b) to generate the second counter electromagnetic field; and
when the first information read/write device (23a) transmits the first electromagnetic field by means of the first signal transmission circuit (26a) and the first antenna module (28a), the controller (27) is further configured to control the second electromagnetic field shielding circuit (25b) to be connected to the first antenna (281) of the second antenna module (28b) to generate the second counter electromagnetic field; or
when the second information read/write device (23b) transmits the second electromagnetic field by means of the second signal transmission circuit (26b) and the second antenna module (28b), the controller (27) is further configured to control the first electromagnetic field shielding circuit (25a) to be connected to the first antenna (281) of the first antenna module (28a) to generate the first counter electromagnetic field.

13. The sample analyzer (100) of any one of claims 6 to 11, **characterized in that** each antenna module (28) is further provided with a second antenna (282), wherein the first electromagnetic field shielding circuit (25a) is capable of being connected to the second antenna (282) of the first antenna module (28a) to generate the first counter electromagnetic field, and the second electromagnetic field shielding circuit (25b) is capable of being connected to the second antenna (282) of the second antenna module (28b) to generate the second counter electromagnetic field; and
when the first information read/write device (23a) transmits the first electromagnetic field by means of the first signal transmission circuit (26a) and the first antenna (281) of the first antenna module (28a), the controller (27) is further configured to control the second electromagnetic field shielding circuit (25b) to be connected to the second antenna (282) of the second antenna module (28b) to generate the second counter electromagnetic field; or
when the second information read/write device (23b) transmits the second electromagnetic field by means of the second signal transmission circuit (26b) and the first antenna (281) of the second antenna module (28b), the controller (27) is further configured to control the first electromagnetic field shielding circuit (25a) to be connected to the second antenna (282) of the first antenna module (28a) to generate the first counter electromagnetic field.

14. A reagent information reading method, applied to a sample analyzer of any one of claims 2 to 13, **characterized in that** the method comprises:
controlling the first information read/write device to transmit the first electromagnetic field by means of the first signal transmission circuit and the first antenna module to the information storage component of the reagent container in the first reagent position, so that the first information read/write device performs signal interaction with the information storage component of the reagent container in the first reagent position; and controlling the second electromagnetic field shielding circuit to be connected to the second antenna module to generate the second counter electromagnetic field, so as to weaken an electromagnetic field transmitted from the first electromagnetic field to the second reagent position;
or
controlling the second information read/write device to transmit the second electromagnetic field by means of the second signal transmission circuit and the second antenna module to the information storage component of the reagent container in the second reagent position, so that the second information read/write device performs signal interaction with the information storage component of the reagent container in the second reagent position; and controlling the first electromagnetic field shielding circuit to be connected to the first antenna module to generate the first counter electromagnetic field, so as to weaken an electromagnetic field transmitted from the second electromagnetic field to the first reagent position.

15. The reagent information reading method of claim 14, **characterized in that** controlling the second electromagnetic field shielding circuit to be connected to the second antenna module to generate the second counter electromagnetic field, so as to weaken an electromagnetic field transmitted from the first electromagnetic field to the second reagent position comprises:
adjusting a current of the second electromagnetic field shielding circuit, so that a direction of the current of the second electromagnetic field shielding circuit is opposite to that of a current of the first signal transmission circuit; or
controlling the first electromagnetic field shielding circuit to be connected to the first antenna module to generate the first counter electromagnetic field, so as to weaken an electromagnetic field transmitted from the second electromagnetic field to the first reagent position comprises:
adjusting a current of the first electromagnetic field shielding circuit, so that a direction of the current of the first electromagnetic field shielding circuit is opposite to that of a current of the second signal transmission circuit.

## Patentansprüche

1. Ein Probenanalysator (100), umfassend:
eine Probenzuführvorrichtung (10), die dazu ausgelegt ist, eine zu untersuchende Blutprobe zuzuführen;
eine Reagenzienzuführvorrichtung (20), die dazu ausgelegt ist, ein Reagenz zur Reaktion mit der zu untersuchenden Blut s zuzuführen;
einen Reaktionsbehälter (30), der dazu ausgelegt ist, die von der Probenzuführvorrichtung (10) zugeführte zu untersuchende Blutprobe und das von der Reagenzzuführvorrichtung (20) zugeführte Reagenz aufzunehmen, sodass die zu untersuchende Blutprobe und das Reagenz miteinander reagieren und eine gemischte Probenlösung bilden; und
eine Testvorrichtung (40), die einen Testbereich (401) aus einem lichtdurchlässigen Material und eine dem Testbereich (401) zugeordnete Lichtquelle (402) umfasst, wobei die Lichtquelle (402) so ausgelegt ist, dass sie die durch den Testbereich (401) fließende gemischte Probenlösung bestrahlt, um den Gehalt eines bestimmten Proteins in der gemischten Probenlösung zu messen;
wobei die Reagenzienzuführvorrichtung (20) umfasst:
ein Reagenzienaufnahmekammer (21), die mit mindestens zwei Reagenzienpositionen (211) versehen ist, wobei die mindestens zwei Reagenzienpositionen (211) jeweils zum Aufnehmen eines ein Reagenz enthaltenden Reagenzbehälters (50) dienen und der Reagenzbehälter (50) mit einer Informationsspeicherkomponente (501) versehen ist, die Reagenzinformationen speichert;
ein Antennenmodul (28);
eine Signalübertragungsschaltung (26), die mit dem Antennenmodul (28) verbindbar ist, um ein elektromagnetisches Sendefeld zu übertragen;
eine Informations-Lese-/Schreibvorrichtung (23), die mit der Signalübertragungsschaltung (26) verbunden und so konfiguriert ist, dass sie in jeder Reagenzposition (211) eine Signalinteraktion mit der Informationsspeicherkomponente (501) des Reagenzbehälters (50) durchführt, um Informationen aus der Informationsspeicherkomponente (501) des Reagenzbehälters (50) auszulesen oder in diese zu schreiben;
sowie
eine Steuereinheit (27), die so ausgelegt ist, dass sie:
die Informations-Lese-/Schreibvorrichtung (23) so zu steuern, dass diese das emittierte elektromagnetische Feld mittels der Signalübertragungsschaltung (26) und des Antennenmoduls (28) aussendet, sodass die Informations-Lese-/Schreibvorrichtung (23) eine Signalinteraktion mit der Informationsspeicherkomponente (211a) des Reagenzbehälters (211) in einer ersten
Reagenzposition (211a) der mindestens zwei Reagenzpositionen (211) eine Signalinteraktion mit der Informationsspeicherkomponente (211a) des Reagenzbehälters (211) durchführt;
**dadurch gekennzeichnet, dass** die Reagenzienzuführvorrichtung (20) ferner umfasst:
eine elektromagnetische Feldabschirmungsschaltung (25), die so ausgelegt ist, dass sie ein gegenläufiges elektromagnetisches
zu erzeugen; und
dass die Steuerung (27) ferner dazu ausgelegt ist,
die elektromagnetische Feldabschirmungsschaltung (25) so zu steuern, dass sie das Gegenfeld erzeugt, um ein elektromagnetisches Feld abzuschwächen, das vom emittierenden elektromagnetischen Feld zu einer zweiten Reagenzposition (211b) der mindestens zwei Reagenzpositionen (211) übertragen wird.

2. Der Probenanalysator (100) nach Anspruch 1, **dadurch gekennzeichnet, dass**,
Das Antennenmodul (28) umfasst ein erstes Antennenmodul (28a), das der ersten Reagenzposition (211a) entspricht, und ein zweites Antennenmodul (28b), das der zweiten Reagenzposition (211b) entspricht, die Signalübertragungsschaltung (26) umfasst eine erste Signalübertragungsschaltung (26a), die der ersten Reagenzposition (211a) entspricht, und eine zweite Signalübertragungsschaltung (26b), die der zweiten Reagenzposition (211b) entspricht, die Informations-Lese-/Schreibvorrichtung (23) umfasst eine erste Informations-Lese-/Schreibvorrichtung (23a), die der ersten Reagenzposition (211a) entspricht, und eine zweite Informations-Lese-/Schreibvorrichtung (23b), die der zweiten Reagenzposition (211b) entspricht, und die Schaltung zur Abschirmung elektromagnetischer Felder (25) umfasst eine erste Schaltung zur Abschirmung elektromagnetischer Felder (25a), die der ersten Reagenzposition (211a) entspricht, und eine zweite Schaltung zur Abschirmung elektromagnetischer Felder (25b), die der zweiten Reagenzposition (211b) entspricht;
die erste Lese-/Schreibvorrichtung (23a) kann über die erste Signalübertragungsschaltung (26a) mit dem ersten Antennenmodul (28a) verbunden werden, um ein erstes elektromagnetisches Feld an die Informationsspeicherkomponente des Reagenzbehälters in der ersten Reagenzposition (211a) zu senden und eine Signalwechselwirkung mit der Informationsspeicherkomponente des Reagenzbehälters in der ersten Reagenzposition (211a) durchzuführen, und die erste Schaltung zur Abschirmung elektromagnetischer Felder (25a) kann mit dem ersten Antennenmodul (28a) verbunden werden, um ein erstes Gegenfeld zu erzeugen;
die zweite Informations-Lese-/Schreibvorrichtung (23b) kann über die zweite Signalübertragungsschaltung (26b) mit dem zweiten Antennenmodul (28b) verbunden werden, um ein zweites elektromagnetisches Feld an die Informationsspeicherkomponente des Reagenzbehälters in der zweiten Reagenzposition (211b) zu senden und eine Signalwechselwirkung mit der Informationsspeicherkomponente des Reagenzbehälters in der zweiten Reagenzposition (211b) durchzuführen, und die zweite Schaltung zur Abschirmung elektromagnetischer Felder (25b) kann mit dem zweiten Antennenmodul (28b) verbunden werden, um ein zweites Gegenfeld zu erzeugen; und die Steuerung (27) ist ferner so konfiguriert, dass sie:
die erste Informations-Lese-/Schreibvorrichtung (23a) so zu steuern, dass sie das erste elektromagnetische Feld mittels der ersten Signalübertragungsschaltung (26a) und des ersten Antennenmoduls (28a) an die Informationsspeicherkomponente des Reagenzbehälters in der ersten Reagenzposition (211a), so dass die erste Informations-Lese-/Schreibvorrichtung (23a) eine Signalwechselwirkung mit der Informationsspeicherkomponente des Reagenzbehälters in der ersten Reagenzposition (211a) durchführt; und die zweite Schaltung zur Abschirmung elektromagnetischer Felder (25b) so zu steuern, dass sie mit dem zweiten Antennenmodul (28b) verbunden wird, um das zweite gegenläufige elektromagnetische Feld zu erzeugen, um ein vom ersten elektromagnetischen Feld zur zweiten Reagenzposition (211b) übertragenes elektromagnetisches Feld abzuschwächen;
oder
die zweite Informations-Lese-/Schreibvorrichtung (23b) so zu steuern, dass sie das zweite elektromagnetische Feld mittels der zweiten Signalübertragungsschaltung (26b) und des zweiten Antennenmoduls (28b) an die Informationsspeicherkomponente des Reagenzbehälters in der zweiten Reagenzposition (211b) überträgt, sodass die zweite Informations-Lese-/Schreibvorrichtung (23b) eine Signalwechselwirkung mit der Informationsspeicherkomponente des Reagenzbehälters in der zweiten Reagenzposition (211b) durchführt; und die erste Schirmungsschaltung (25a) für das elektromagnetische Feld so zu steuern, dass sie mit dem ersten Antennenmodul (28a) in n Verbindung steht, um das erste Gegenfeld zu erzeugen, um so ein vom zweiten elektromagnetischen Feld zur ersten Reagenzposition (211a) übertragenes elektromagnetisches Feld abzuschwächen.

3. Der Probenanalysator (100) nach Anspruch 2, **dadurch gekennzeichnet, dass** die Feldrichtung des zweiten Gegen-Elektromagnetfelds, das von der zweiten elektromagnetischen Feldabschirmungsschaltung (25b) erzeugt wird, entgegengesetzt zu der des ersten elektromagnetischen Felds ist, das von der ersten Informations-Lese-/Schreibvorrichtung (23a) über die erste Signalübertragungsschaltung (26a) und das erste Antennenmodul (28a) übertragen wird; oder
die Feldrichtung des ersten gegenläufigen elektromagnetischen Feldes, das von der ersten elektromagnetischen Feldabschirmungsschaltung (25a) erzeugt wird, entgegengesetzt zu der des zweiten elektromagnetischen Feldes ist, das von der zweiten Informations-Lese-/Schreibvorrichtung (23b) über die zweite Signalübertragungsschaltung (26b) und das zweite Antennenmodul (28b) übertragen wird.

4. Der Probenanalysator (100) nach Anspruch 3, **dadurch gekennzeichnet, dass** eine Feldstärke des zweiten Gegenfeldes, das von der zweiten elektromagnetischen Feldabschirmungsschaltung (25b) erzeugt wird, größer oder gleich einer Feldstärke des ersten elektromagnetischen Feldes ist, das von der ersten Informations-Lese-/Schreibvorrichtung (23a) über die erste Signalübertragungsschaltung (26a) und das erste Antennenmodul (28a) übertragen wird; oder
die Feldstärke des ersten gegenläufigen elektromagnetischen Feldes, das von der ersten elektromagnetischen Feldabschirmungsschaltung (25a) erzeugt wird, größer oder gleich der Feldstärke des zweiten elektromagnetischen Feldes ist, das von der zweiten Informations-Lese-/Schreibvorrichtung (23b) über die zweite Signalübertragungsschaltung (26b) und das zweite Antennenmodul (28b) übertragen wird.

5. Der Probenanalysator (100) nach einem der Ansprüche 2 bis 4, **dadurch gekennzeichnet, dass** die erste Signalübertragungsschaltung (26a) und die zweite Signalübertragungsschaltung (26b) voneinander unabhängig sind; und/oder die erste elektromagnetische Feldabschirmungsschaltung (25a) und die zweite elektromagnetische Feldabschirmungsschaltung (25b) voneinander unabhängig sind; und/oder die erste Informations-Lese-/Schreibvorrichtung (23a) und die zweite Informations-Lese-/Schreibvorrichtung (23b) voneinander unabhängig sind.

6. Der Probenanalysator (100) nach einem der Ansprüche 2 bis 5, **dadurch gekennzeichnet, dass** jede Signalübertragungsschaltung (26) eine erste Anpassungsschaltung (261) umfasst und jedes Antennenmodul (28) mit einer ersten Antenne (281) versehen ist;
wobei die erste Informations-Lese-/Schreibvorrichtung (23a) mit der ersten Anpassungsschaltung (261) der ersten Signalübertragungsschaltung (26a) verbunden ist und die erste Anpassungsschaltung (261) der ersten Signalübertragungsschaltung (26a) mit der ersten Antenne (281) des ersten Antennenmoduls (28a) verbunden werden kann, um mittels der ersten Anpassungsschaltung (261) der ersten Signalübertragungsschaltung (26a) und der ersten Antenne (281) des ersten Antennenmoduls (28a) eine Signalinteraktion mit der Informationsspeicherkomponente des Reagenzbehälters in der ersten Reagenzposition (211a) durchzuführen; und
wobei die zweite Informations-Lese-/Schreibvorrichtung (23b) mit der ersten Anpassungsschaltung (261) der zweiten Signalübertragungsschaltung (26b) verbunden ist und die erste Anpassungsschaltung (261) der zweiten Signalübertragungsschaltung (26b) mit der ersten Antenne (281) des zweiten Antennenmoduls (28b) verbunden werden kann, um mittels der ersten Anpassungsschaltung (261) der zweiten Signalübertragungsschaltung (26b) und der ersten Antenne (281) des zweiten Antennenmoduls (28b) eine Signalinteraktion mit der Informationsspeicherkomponente des Reagenzbehälters in der zweiten Reagenzposition durchzuführen.

7. Der Probenanalysator (100) nach Anspruch 6, **dadurch gekennzeichnet, dass** jede elektromagnetische Feldabschirmungsschaltung (25) eine Abschirmschleife (251) umfasst; und
wenn die erste Informations-Lese-/Schreibvorrichtung (23a) das erste elektromagnetische Feld mittels der ersten Signalübertragungsschaltung (26a) und des ersten Antennenmoduls (28a) aussendet, die Steuereinheit (27) ferner so ausgelegt ist, dass sie einen Strom der Abschirmschleife (251) der zweiten elektromagnetischen Feldabschirmungsschaltung (25b) so steuert, dass er größer ist als ein voreingestellter Stromwert, und die Richtung dieses Stroms entgegengesetzt zu der eines Stroms der ersten Signalübertragungsschaltung (26a) ist; oder
wenn die zweite Informations-Lese-/Schreibvorrichtung (23b) das zweite elektromagnetische Feld mittels der zweiten Signalübertragungsschaltung (26b) und des zweiten Antennenmoduls (28b) aussendet, ist die Steuereinheit (27) ferner so ausgelegt, dass sie einen Strom der Abschirmschleife (251) der ersten elektromagnetischen Feld-Abschirmschaltung (25a) so steuert, dass er größer als ein voreingestellter Stromwert ist, und dass die Richtung dieses Stroms entgegengesetzt zu der eines Stroms der zweiten Signalübertragungsschaltung (26b) ist.

8. Der Probenanalysator (100) nach Anspruch 7, **dadurch gekennzeichnet, dass** jede Informations-Lese-/Schreibvorrichtung (23) ferner so ausgelegt ist, dass sie ein Abschirmsignal ausgibt, um den Strom der entsprechenden Abschirmschleife (251) einzustellen, und dass ein Abschirmsignal-Ausgangsanschluss ( ) jeder Informations-Lese-/Schreibvorrichtung (23) elektrisch mit der entsprechenden Abschirmschleife (251) verbunden ist; und
wenn die erste Informations-Lese-/Schreibvorrichtung (23a) das erste elektromagnetische Feld mittels der ersten Signalübertragungsschaltung (26a) und des ersten Antennenmoduls (28a) aussendet, ist die Steuereinheit (27) ferner so ausgelegt, dass sie die zweite Informations-Lese-/Schreibvorrichtung (23b) so steuert, dass diese ein Abschirmsignal ausgibt, sodass der Strom der Abschirmschleife (251) der zweiten elektromagnetischen Feldabschirmungsschaltung (25b) größer ist als der voreingestellte Stromwert und die Richtung dieses Stroms entgegengesetzt zu der des Stroms der ersten Signalübertragungsschaltung (26a) ist;
oder wenn die zweite Informations-Lese-/Schreibvorrichtung (23b) das zweite elektromagnetische Feld mittels der zweiten Signalübertragungsschaltung (26b) und des zweiten Antennenmoduls (28b) überträgt, ist die Steuereinheit (27) ferner so ausgelegt, dass sie die erste Informations-Lese-/Schreibvorrichtung (23a) so steuert, dass diese ein Abschirmsignal ausgibt, sodass der Strom der Abschirmschleife (251) der ersten elektromagnetischen Feld-Abschirmschaltung (25a) größer ist als der voreingestellte Stromwert und die Richtung dieses Stroms entgegengesetzt zu der des Stroms der zweiten Signalübertragungsschaltung (26b) ist.

9. Der Probenanalysator (100) nach Anspruch 7, **dadurch gekennzeichnet, dass** jede Abschirmschleife (251) eine zweite Anpassungsschaltung (2511) und ein Schaltmodul (2512) umfasst, wobei die Impedanz der zweiten Anpassungsschaltung (2511) geringer ist als die der ersten Anpassungsschaltung; und
Wenn die erste Lese-/Schreibvorrichtung (23a) das erste elektromagnetische Feld mittels der ersten Signalübertragungsschaltung (26a) und des ersten Antennenmoduls (28a) aussendet, ist die Steuereinheit (27) ferner so ausgelegt, dass sie das Schaltmodul (2512) der Abschirmschleife (251) der zweiten elektromagnetischen Feldabschirmschaltung (25b) so zu steuern, dass die zweite Anpassungsschaltung (2511) der Abschirmschleife (251) der zweiten elektromagnetischen Feldabschirmschaltung (25b) mit dem zweiten Antennenmodul (28b) verbunden wird, sodass die zweite Anpassungsschaltung (2511) der Abschirmschleife (251) der zweiten elektromagnetischen Feldabschirmschaltung (25b) und das zweite Antennenmodul (28b) eine geschlossene Schleife bilden, wobei die Impedanz der Abschirmschleife (251) der zweiten elektromagnetischen Feldabschirmschaltung (25b) geringer ist als die der ersten Signalübertragungsschaltung (26a); oder
wenn die zweite Informations-Lese-/Schreibvorrichtung (23b) das zweite elektromagnetische Feld mittels der zweiten Signalübertragungsschaltung (26b) und des zweiten Antennenmoduls (28b) überträgt, ist die Steuereinheit (27) ferner so ausgelegt, dass sie das Schaltmodul (2512) der Abschirmschleife (251) der ersten elektromagnetischen Feldabschirmschaltung (25a) so steuert, dass die zweite Anpassungsschaltung (2511) der Abschirmschleife (251) der ersten elektromagnetischen Feldabschirmschaltung (25a) mit dem ersten Antennenmodul (28a) verbunden wird, sodass die zweite Anpassungsschaltung (2511) der Abschirmschleife (251) der ersten elektromagnetischen Feldabschirmungsschaltung (25a) und das erste Antennenmodul (28a) eine geschlossene Schleife bilden, wobei die Impedanz der Abschirmschleife (251) der ersten elektromagnetischen Feldabschirmungsschaltung (25a) geringer ist als die der zweiten Signalübertragungsschaltung (26b).

10. Der Probenanalysator (100) nach Anspruch 7, **dadurch gekennzeichnet, dass** jede Abschirmschleife (251) ein Schaltmodul (2512) umfasst; und
wenn die erste Informations-Lese-/Schreibvorrichtung (23a) das erste elektromagnetische Feld mittels der ersten Signalübertragungsschaltung (26a) und des ersten Antennenmoduls (28a) überträgt, die Steuerung (27) ferner so konfiguriert ist, dass sie das Schaltmodul (2512) der Abschirmschleife (251) der zweiten elektromagnetischen Feldabschirmungsschaltung (25b) so steuert, dass diese geschlossen wird, sodass das Schaltmodul (2512) der Abschirmschleife (251) der zweiten elektromagnetischen Feldabschirmschaltung (25b) und das zweite Antennenmodul (28b) eine geschlossene Schleife bilden, wobei die Impedanz der Abschirmschleife (251) der zweiten elektromagnetischen Feldabschirmschaltung (25b) geringer ist als die der ersten Signalübertragungsschaltung (26a); oder
wenn die zweite Informations-Lese-/Schreibvorrichtung (23b) das zweite elektromagnetische Feld mittels der zweiten Signalübertragungsschaltung (26b) und des zweiten Antennenmoduls (28b) überträgt, ist die Steuerung (27) ferner so ausgelegt, dass sie das Schaltmodul (2512) der Abschirmschleife (251) der ersten elektromagnetischen Feldabschirmschaltung (25a) so steuert, dass es geschlossen wird, sodass das Schaltmodul (2512) der Abschirmschleife (251) der ersten elektromagnetischen Feldabschirmungsschaltung (25a) und das erste Antennenmodul (28a) eine geschlossene Schleife bilden, wobei die Impedanz der Abschirmschleife (251) der ersten elektromagnetischen Feldabschirmungsschaltung (25a) geringer ist als die der zweiten Signalübertragungsschaltung (26b) ist.

11. Der Probenanalysator (100) nach Anspruch 7, **dadurch gekennzeichnet, dass** jede Abschirmschleife (251) eine zweite Anpassungsschaltung (2511) und ein Schaltmodul (2512) umfasst; und
Wenn das erste Lese-/Schreibgerät (23a) das erste elektromagnetische Feld mittels der ersten Signalübertragungsschaltung (26a) und des ersten Antennenmoduls (28a) überträgt, ist die Steuerungs (27) ferner so ausgelegt, dass sie das Schaltmodul (2512) der Abschirmschleife (251) der zweiten elektromagnetischen Feldabschirmschaltung (25b) so steuert, dass die zweite Anpassungsschaltung (2511) der Abschirmschleife (251) der zweiten elektromagnetischen Feld-Abschirmschaltung (25b) mit dem zweiten Antennenmodul (28b) zu verbinden, das Schaltmodul (2512) der Abschirmschleife (251) der zweiten elektromagnetischen Feld-Abschirmschaltung (25b) so zu steuern, dass die zweite Anpassungsschaltung (2511) der Abschirmschleife (251) der zweiten elektromagnetischen Feldabschirmungsschaltung (25b) mit der zweiten Informations-Lese-/Schreibvorrichtung (23b) zu verbinden, und die zweite Informations-Lese-/Schreibvorrichtung (23b) so zu steuern, dass sie ein Abschirmsignal an die zweite elektromagnetische Feldabschirmungsschaltung (25b) ausgibt; oder
wenn die zweite Informations-Lese-/Schreibvorrichtung (23b) das zweite elektromagnetische Feld mittels der zweiten Signalübertragungsschaltung (26b) und des zweiten Antennenmoduls (28b) aussendet, ist die Steuerung (27) ferner so ausgelegt, dass sie das Schaltmodul (2512) der Abschirmschleife (251) der ersten elektromagnetischen Feld-Abschirmschaltung (25a) so steuert, dass die zweite Anpassungsschaltung (2511) der Abschirmschleife (251) der ersten elektromagnetischen Feld-Abschirmschaltung (25a) mit dem ersten Antennenmodul (28a) zu verbinden, das Schaltmodul (2512) der Abschirmschleife (251) der ersten elektromagnetischen Feld-Abschirmschaltung (25a) so zu steuern, dass die zweite Anpassungsschaltung (2511) der Abschirmschleife (251) der ersten elektromagnetischen Feldabschirmungsschaltung (25a) mit der ersten Informations-Lese-/Schreibvorrichtung (23a) zu verbinden, und die erste Informations-Lese-/Schreibvorrichtung (23a) so zu steuern, dass sie ein Abschirmsignal an die erste elektromagnetische Feldabschirmungsschaltung (25a) ausgibt.

12. Der Probenanalysator (100) nach einem der Ansprüche 6 bis 11, **dadurch gekennzeichnet, dass** jede erste Antenne (281) ferner dazu ausgelegt ist, das Gegen-Elektromagnetfeld zu senden, wobei die erste elektromagnetische Feldabschirmschaltung (25a) mit der ersten Antenne (281) des ersten Antennenmoduls (28a) angeschlossen werden kann, um das erste Gegen-Elektromagnetfeld zu erzeugen, und die zweite Schaltung zur Abschirmung elektromagnetischer Felder (25b) an die erste Antenne (281) des zweiten Antennenmoduls (28b) angeschlossen werden kann, um das zweite Gegen-Elektromagnetfeld zu erzeugen; und
wenn die erste Informations-Lese-/Schreibvorrichtung (23a) das erste elektromagnetische Feld mittels der ersten Signalübertragungsschaltung (26a) und des ersten Antennenmoduls (28a) aussendet, ist die Steuereinheit (27) ferner so konfiguriert, dass sie die zweite Schaltung zur Abschirmung elektromagnetischer Felder (25b) so steuert, dass diese mit der ersten Antenne (281) des zweiten Antennenmoduls (28b) verbunden wird, um das zweite gegenläufige elektromagnetische Feld zu erzeugen; oder
wenn das zweite Lese-/Schreibgerät (23b) das zweite elektromagnetische Feld mittels der zweiten Signalübertragungsschaltung (26b) und des zweiten Antennenmoduls (28b) aussendet, ist die Steuereinheit (27) ferner so ausgelegt, dass sie die erste Schaltung zur Abschirmung elektromagnetischer Felder (25a) so steuert, dass diese mit der ersten Antenne (281) des ersten Antennenmoduls (28a) verbunden wird, um das erste Gegenfeld zu erzeugen.

13. Der Probenanalysator (100) nach einem der Ansprüche 6 bis 11, **dadurch gekennzeichnet, dass** jedes Antennenmodul (28) ferner mit einer zweiten Antenne (282) versehen ist, wobei die erste Schaltung zur Abschirmung elektromagnetischer Felder (25a) mit der zweiten Antenne (282) des ersten Antennenmoduls (28a) angeschlossen werden kann, um das erste gegenläufige elektromagnetische Feld zu erzeugen, und die zweite elektromagnetische Feldabschirmungsschaltung (25b) mit der zweiten Antenne (282) des zweiten Antennenmoduls (28b) verbunden werden kann, um das zweite gegenläufige elektromagnetische Feld zu erzeugen; und
wenn die erste Lese-/Schreibvorrichtung (23a) das erste elektromagnetische Feld mittels der ersten Signalübertragungsschaltung (26a) und der ersten Antenne (281) des ersten Antennenmoduls (28a) aussendet, ist die Steuereinheit (27) ferner so ausgelegt, dass sie die zweite Schaltung zur Abschirmung elektromagnetischer Felder (25b) so steuert, dass diese mit der zweiten Antenne (282) des zweiten Antennenmoduls (28b) verbunden wird, um das zweite gegenläufige elektromagnetische Feld zu erzeugen; oder
wenn die zweite Informations-Lese-/Schreibvorrichtung (23b) das zweite elektromagnetische Feld mittels der zweiten Signalübertragungsschaltung (26b) und der ersten Antenne (281) des zweiten Antennenmoduls (28b) aussendet, ist die Steuereinheit (27) ferner so ausgelegt, dass sie die erste Schaltung zur Abschirmung elektromagnetischer Felder (25a) so steuert, dass diese mit der zweiten Antenne (282) des ersten Antennenmoduls (28a) verbunden wird, um das erste Gegenfeld zu erzeugen.

14. Verfahren zum Auslesen von Reagenzieninformationen, anwendbar auf einen Probenanalysator gemäß einem der Ansprüche 2 bis 13, **dadurch gekennzeichnet, dass** das Verfahren umfasst:
das Steuern der ersten Informations-Lese-/Schreibvorrichtung, um das erste elektromagnetische Feld mittels der ersten Signalübertragungsschaltung und des ersten Antennenmoduls an die Informationsspeicherkomponente des Reagenzbehälters in der ersten Reagenzposition zu senden, so dass die erste Informations-Lese-/Schreibvorrichtung eine Signalwechselwirkung mit der Informationsspeicherkomponente des Reagenzbehälters in der ersten Reagenzposition durchführt; und das Ansteuern der zweiten elektromagnetischen Feldabschirmungsschaltung, die mit dem zweiten Antennenmodul verbunden ist, um das zweite gegenläufige elektromagnetische Feld zu erzeugen, um ein vom ersten elektromagnetischen Feld zur zweiten Reagenzposition übertragenes elektromagnetisches Feld abzuschwächen;
oder
Steuerung der zweiten Informations-Lese-/Schreibvorrichtung, um das zweite elektromagnetische Feld mittels der zweiten Signalübertragungsschaltung und des zweiten Antennenmoduls an die Informationsspeicherkomponente des Reagenzbehälters in der zweiten Reagenzposition zu übertragen, sodass die zweite Informations-Lese-/Schreibvorrichtung eine Signalwechselwirkung mit der Informationsspeicherkomponente des Reagenzbehälters in der zweiten Reagenzposition durchführt; und Ansteuern der ersten elektromagnetischen Feldabschirmungsschaltung, die mit dem ersten Antennenmodul verbunden ist, um das erste Gegenfeld zu erzeugen, um ein elektromagnetisches Feld abzuschwächen, das vom zweiten elektromagnetischen Feld zur ersten Reagenzposition übertragen wird.

15. Verfahren zum Auslesen von Reagenzieninformationen nach Anspruch 14, **dadurch gekennzeichnet, dass** das Steuern der zweiten elektromagnetischen Feldabschirmungsschaltung, die mit dem zweiten Antennenmodul verbunden ist, zur Erzeugung des zweiten Gegenfeldes, um ein vom ersten elektromagnetischen Feld zur zweiten Reagenzposition übertragenes elektromagnetisches Feld abzuschwächen, Folgendes umfasst:
das Einstellen eines Stroms der zweiten elektromagnetischen Feldabschirmungsschaltung, sodass die Richtung des Stroms der zweiten elektromagnetischen Feldabschirmungsschaltung entgegengesetzt zu der eines Stroms der ersten Signalübertragungsschaltung ist; oder
die Steuerung der ersten elektromagnetischen Feldabschirmungsschaltung, die mit dem ersten Antennenmodul verbunden ist, zur Erzeugung des ersten Gegenfeldes, um ein vom zweiten elektromagnetischen Feld zur ersten Reagenzposition übertragenes elektromagnetisches Feld abzuschwächen, umfasst:
das Einstellen eines Stroms der ersten elektromagnetischen Feldabschirmungsschaltung, sodass die Richtung des Stroms der ersten elektromagnetischen Feldabschirmungsschaltung entgegengesetzt zu der des Stroms der zweiten Signalübertragungsschaltung ist.

## Revendications

1. Analyseur d'échantillons (100), comprenant :
un dispositif de fourniture d'échantillon (10) configuré pour fournir un échantillon de sang à analyser ;
un dispositif de fourniture de réactif (20) configuré pour fournir un réactif destiné à réagir avec l'échantillon de sang à analyser ;
un contenant de réaction (30) configuré pour recevoir l'échantillon de sang à analyser qui est fourni par le dispositif de fourniture d'échantillon (10) et le réactif qui est fourni par dispositif de fourniture de réactif (20), de sorte que l'échantillon de sang à analyser et le réactif réagissent pour former une solution d'échantillon mélangé ; et
un dispositif d'analyse (40) comprenant une zone d'analyse (401) constituée d'un matériau transmettant la lumière et une source de lumière (402) correspondant à la zone d'analyse (401), la source de lumière (402) étant configurée pour irradier la solution d'échantillon mélangé s'écoulant à travers la zone d'analyse (401), de sorte à mesurer une teneur d'une protéine spécifique dans la solution d'échantillon mélangé ;
où le dispositif de fourniture de réactif (20) comprend :
un compartiment de disposition de réactif (21) doté d'au moins deux positions de réactif (211), où les au moins deux positions de réactif (211) sont utilisées pour respectivement disposer un contenant de réactif (50) contenant un réactif, et le contenant de réactif (50) est doté d'un composant de stockage d'informations (501) stockant des informations sur le réactif ;
un module d'antenne (28) ;
un circuit de transmission de signal (26) pouvant être relié au module d'antenne (28) pour transmettre un champ électromagnétique d'émission ;
un dispositif d'écriture/de lecture d'informations (23) relié au circuit de transmission de signal (26) et configuré pour réaliser une interaction de signal avec le composant de stockage d'informations (501) du contenant de réactif (50) dans chaque position de réactif (211), de sorte à lire ou écrire des informations depuis ou sur le composant de stockage d'informations (501) du contenant de réactif (50) ;
et
une unité de commande (27) configurée pour :
commander au dispositif d'écriture/de lecture d'informations (23) de transmettre le champ électromagnétique d'émission au moyen du circuit de transmission de signal (26) et du module d'antenne (28), de sorte que le dispositif d'écriture/de lecture d'informations (23) réalise une interaction de signal avec le composant de stockage d'informations (501) du contenant de réactif (50) dans une première position de réactif (211a) parmi les au moins deux positions de réactif (211) ;
**caractérisé en ce que** le dispositif de fourniture de réactif (20) comprend en outre :
un circuit de blindage électromagnétique (25) configuré pour générer un contre-champ électromagnétique ; et
**en ce que** l'unité de commande (27) est en outre configurée pour :
commander au circuit de blindage électromagnétique (25) de générer le contre-champ électromagnétique, de sorte à affaiblir un champ électromagnétique transmis par le champ électromagnétique d'émission à une seconde position de réactif (211b) parmi les au moins deux positions de réactif (211).

2. Analyseur d'échantillons (100) selon la revendication 1, **caractérisé en ce que**,
le module d'antenne (28) comprend un premier module d'antenne (28a) correspondant à la première position de réactif (211a) et un second module d'antenne (28b) correspondant à la seconde position de réactif (211b), le circuit de transmission de signal (26) comprend un premier circuit de transmission de signal (26a) correspondant à la première position de réactif (211a) et un second circuit de transmission de signal (26b) correspondant à la seconde position de réactif (211b), le dispositif d'écriture/de lecture d'informations (23) comprend un premier dispositif d'écriture/de lecture d'informations (23a) correspondant à la première position de réactif (211a) et un second dispositif d'écriture/de lecture d'informations (23b) correspondant à la seconde position de réactif (211b), et le circuit de blindage électromagnétique (25) comprend un premier circuit de blindage électromagnétique (25a) correspondant à la première position de réactif (211a) et un second circuit de blindage électromagnétique (25b) correspondant à la seconde position de réactif (211b) ;
le premier dispositif d'écriture/de lecture d'informations (23a) peut être relié au premier module d'antenne (28a) via le premier circuit de transmission de signal (26a), de sorte à transmettre un premier champ électromagnétique au composant de stockage d'informations du contenant de réactif dans la première position de réactif (211a) et réaliser une interaction de signal avec le composant de stockage d'informations du contenant de réactif dans la première position de réactif (211a), et le premier circuit de blindage électromagnétique (25a) peut être relié au premier module d'antenne (28a) pour générer un premier contre-champ électromagnétique ;
le second dispositif d'écriture/de lecture d'informations (23b) peut être relié au second module d'antenne (28b) via le second circuit de transmission de signal (26b), de sorte à transmettre un second champ électromagnétique au composant de stockage d'informations du contenant de réactif dans la seconde position de réactif (211b) et réaliser une interaction de signal avec le composant de stockage d'informations du contenant de réactif dans la seconde position de réactif (211b), et le second circuit de blindage électromagnétique (25b) peut être relié au second module d'antenne (28b) pour générer un second contre-champ électromagnétique ; et
l'unité de commande (27) est en outre configurée pour :
commander au premier dispositif d'écriture/de lecture d'informations (23a) de transmettre le premier champ électromagnétique au moyen du premier circuit de transmission de signal (26a) et du premier module d'antenne (28a) au composant de stockage d'informations du contenant de réactif dans la première position de réactif (211a), de sorte que le premier dispositif d'écriture/de lecture d'informations (23a) réalise une interaction de signal avec le composant de stockage d'informations du contenant de réactif dans la première position de réactif (211a) ; et commander au second circuit de blindage électromagnétique (25b) de se relier au second module d'antenne (28b) pour générer le second contre-champ électromagnétique, de sorte à affaiblir un champ électromagnétique transmis par le premier champ électromagnétique à la seconde position de réactif (211b) ;
ou
commander au second dispositif d'écriture/de lecture d'informations (23b) de transmettre le second champ électromagnétique au moyen du second circuit de transmission de signal (26b) et du second module d'antenne (28b) au composant de stockage d'informations du contenant de réactif dans la seconde position de réactif (211b), de sorte que le second dispositif d'écriture/de lecture d'informations (23b) réalise une interaction de signal avec le composant de stockage d'informations du contenant de réactif dans la seconde position de réactif (211b) ; et commander au premier circuit de blindage électromagnétique (25a) de se relier au premier module d'antenne (28a) pour générer le premier contre-champ électromagnétique, de sorte à affaiblir un champ électromagnétique transmis par le second champ électromagnétique à la première position de réactif (211a).

3. Analyseur d'échantillons (100) selon la revendication 2, **caractérisé en ce qu'**une direction de champ du second contre-champ électromagnétique généré par le second circuit de blindage électromagnétique (25b) est opposée à celle du premier champ électromagnétique transmis par le premier dispositif d'écriture/de lecture d'informations (23a) via le premier circuit de transmission de signal (26a) et le premier module d'antenne (28a) ; ou
une direction de champ du premier contre-champ électromagnétique généré par le premier circuit de blindage électromagnétique (25a) est opposée à celle du second champ électromagnétique transmis par le second dispositif d'écriture/de lecture d'informations (23b) via le second circuit de transmission de signal (26b) et le second module d'antenne (28b).

4. Analyseur d'échantillons (100) selon la revendication 3, **caractérisé en ce qu'**une force de champ du second contre-champ électromagnétique généré par le second circuit de blindage électromagnétique (25b) est supérieure ou égale à une force de champ du premier champ électromagnétique transmis par le premier dispositif d'écriture/de lecture d'informations (23a) via le premier circuit de transmission de signal (26a) et le premier module d'antenne (28a) ; ou
une force de champ du premier contre-champ électromagnétique généré par le premier circuit de blindage électromagnétique (25a) est supérieure ou égale à une force de champ du second champ électromagnétique transmis par le second dispositif d'écriture/de lecture d'informations (23b) via le second circuit de transmission de signal (26b) et le second module d'antenne (28b).

5. Analyseur d'échantillons (100) selon l'une quelconque des revendications 2 à 4, **caractérisé en ce que** le premier circuit de transmission de signal (26a) et le second circuit de transmission de signal (26b) sont indépendants l'un de l'autre ; et/ou le premier circuit de blindage électromagnétique (25a) et le second circuit de blindage électromagnétique (25b) sont indépendants l'un de l'autre ; et/ou le premier dispositif d'écriture/de lecture d'informations (23a) et le second dispositif d'écriture/de lecture d'informations (23b) sont indépendants l'un de l'autre.

6. Analyseur d'échantillons (100) selon l'une quelconque des revendications 2 à 5, **caractérisé en ce que**
chaque circuit de transmission de signal (26) comprend un premier circuit d'appariement (261), et chaque module d'antenne (28) est doté d'une première antenne (281) ;
où le premier dispositif d'écriture/de lecture d'informations (23a) est relié au premier circuit d'appariement (261) du premier circuit de transmission de signal (26a), et le premier circuit d'appariement (261) du premier circuit de transmission de signal (26a) peut être relié à la première antenne (281) du premier module d'antenne (28a), de sorte à réaliser une interaction de signal avec le composant de stockage d'informations du contenant de réactif dans la première position de réactif (211a) au moyen du premier circuit d'appariement (261) du premier circuit de transmission de signal (26a) et de la première antenne (281) du premier module d'antenne (28a) ; et
où le second dispositif d'écriture/de lecture d'informations (23b) est relié au premier circuit d'appariement (261) du second circuit de transmission de signal (26b), et le premier circuit d'appariement (261) du second circuit de transmission de signal (26b) peut être relié à la première antenne (281) du second module d'antenne (28b), de sorte à réaliser une interaction de signal avec le composant de stockage d'informations du contenant de réactif dans la seconde position de réactif au moyen du premier circuit d'appariement (261) du second circuit de transmission de signal (26b) et de la première antenne (281) du second module d'antenne (28b).

7. Analyseur d'échantillons (100) selon la revendication 6, **caractérisé en ce que** chaque circuit de blindage électromagnétique (25) comprend une boucle de blindage (251) ; et
lorsque le premier dispositif d'écriture/de lecture d'informations (23a) transmet le premier champ électromagnétique au moyen du premier circuit de transmission de signal (26a) et du premier module d'antenne (28a), l'unité de commande (27) est en outre configurée pour commander un courant de la boucle de blindage (251) du second circuit de blindage électromagnétique (25b) de sorte à ce qu'il soit supérieur à une valeur de courant prédéfinie, et un sens dudit courant de sorte à ce qu'il soit opposé à celui d'un courant du premier circuit de transmission de signal (26a) ; ou
lorsque le second dispositif d'écriture/de lecture d'informations (23b) transmet le second champ électromagnétique au moyen du second circuit de transmission de signal (26b) et du second module d'antenne (28b), l'unité de commande (27) est en outre configurée pour commander un courant de la boucle de blindage (251) du premier circuit de blindage électromagnétique (25a) de sorte à ce qu'il soit supérieur à une valeur de courant prédéfinie, et un sens dudit courant de sorte à ce qu'il soit opposé à celui d'un courant du second circuit de transmission de signal (26b).

8. Analyseur d'échantillons (100) selon la revendication 7, **caractérisé en ce que** chaque dispositif d'écriture/de lecture d'informations (23) est en outre configuré pour fournir un signal de blindage afin de régler le courant de la boucle de blindage correspondante (251), et une borne de sortie de signal de blindage de chaque dispositif d'écriture/de lecture d'informations (23) est électriquement reliée à la boucle de blindage correspondante (251) ; et
lorsque le premier dispositif d'écriture/de lecture d'informations (23a) transmet le premier champ électromagnétique au moyen du premier circuit de transmission de signal (26a) et du premier module d'antenne (28a), l'unité de commande (27) est en outre configurée pour commander au second dispositif d'écriture/de lecture d'informations (23b) de fournir un signal de blindage, de sorte que le courant de la boucle de blindage (251) du second circuit de blindage électromagnétique (25b) est supérieur à la valeur de courant prédéfinie, et le sens dudit courant est opposé à celui du courant du premier circuit de transmission de signal (26a) ;
ou lorsque le second dispositif d'écriture/de lecture d'informations (23b) transmet le second champ électromagnétique au moyen du second circuit de transmission de signal (26b) et du second module d'antenne (28b), l'unité de commande (27) est en outre configurée pour commander au premier dispositif d'écriture/de lecture d'informations (23a) de fournir un signal de blindage, de sorte que le courant de la boucle de blindage (251) du premier circuit de blindage électromagnétique (25a) est supérieur à la valeur de courant prédéfinie, et le sens dudit courant est opposé à celui du courant du second circuit de transmission de signal (26b).

9. Analyseur d'échantillons (100) selon la revendication 7, **caractérisé en ce que** chaque boucle de blindage (251) comprend un second circuit d'appariement (2511) et un module de commutation (2512), où une impédance du second circuit d'appariement (2511) est inférieure à celle du premier circuit d'appariement ; et
lorsque le premier dispositif d'écriture/de lecture d'informations (23a) transmet le premier champ électromagnétique au moyen du premier circuit de transmission de signal (26a) et du premier module d'antenne (28a), l'unité de commande (27) est en outre configurée pour commander au module de commutation (2512) de la boucle de blindage (251) du second circuit de blindage électromagnétique (25b) de relier le second circuit d'appariement (2511) de la boucle de blindage (251) du second circuit de blindage électromagnétique (25b) au second module d'antenne (28b), de sorte que le second circuit d'appariement (2511) de la boucle de blindage (251) du second circuit de blindage électromagnétique (25b) et le second module d'antenne (28b) forment une boucle fermée, où une impédance de la boucle de blindage (251) du second circuit de blindage électromagnétique (25b) est inférieure à celle du premier circuit de transmission de signal (26a) ; ou
lorsque le second dispositif d'écriture/de lecture d'informations (23b) transmet le second champ électromagnétique au moyen du second circuit de transmission de signal (26b) et du second module d'antenne (28b), l'unité de commande (27) est en outre configurée pour commander au module de commutation (2512) de la boucle de blindage (251) du premier circuit de blindage électromagnétique (25a) de relier le second circuit d'appariement (2511) de la boucle de blindage (251) du premier circuit de blindage électromagnétique (25a) au premier module d'antenne (28a), de sorte que le second circuit d'appariement (2511) de la boucle de blindage (251) du premier circuit de blindage électromagnétique (25a) et le premier module d'antenne (28a) forment une boucle fermée, où une impédance de la boucle de blindage (251) du premier circuit de blindage électromagnétique (25a) est inférieure à celle du second circuit de transmission de signal (26b).

10. Analyseur d'échantillons (100) selon la revendication 7, **caractérisé en ce que** chaque boucle de blindage (251) comprend un module de commutation (2512) ; et
lorsque le premier dispositif d'écriture/de lecture d'informations (23a) transmet le premier champ électromagnétique au moyen du premier circuit de transmission de signal (26a) et du premier module d'antenne (28a), l'unité de commande (27) est en outre configurée pour commander au module de commutation (2512) de la boucle de blindage (251) du second circuit de blindage électromagnétique (25b) de se fermer, de sorte que le module de commutation (2512) de la boucle de blindage (251) du second circuit de blindage électromagnétique (25b) et le second module d'antenne (28b) forment une boucle fermée, où une impédance de la boucle de blindage (251) du second circuit de blindage électromagnétique (25b) est inférieure à celle du premier circuit de transmission de signal (26a) ; ou
lorsque le second dispositif d'écriture/de lecture d'informations (23b) transmet le second champ électromagnétique au moyen du second circuit de transmission de signal (26b) et du second module d'antenne (28b), l'unité de commande (27) est en outre configurée pour commander au module de commutation (2512) de la boucle de blindage (251) du premier circuit de blindage électromagnétique (25a) de se fermer, de sorte que le module de commutation (2512) de la boucle de blindage (251) du premier circuit de blindage électromagnétique (25a) et le premier module d'antenne (28a) forment une boucle fermée, où une impédance de la boucle de blindage (251) du premier circuit de blindage électromagnétique (25a) est inférieure à celle du second circuit de transmission de signal (26b).

11. Analyseur d'échantillons (100) selon la revendication 7, **caractérisé en ce que** chaque boucle de blindage (251) comprend un second circuit d'appariement (2511) et un module de commutation (2512) ; et
lorsque le premier dispositif d'écriture/de lecture d'informations (23a) transmet le premier champ électromagnétique au moyen du premier circuit de transmission de signal (26a) et du premier module d'antenne (28a), l'unité de commande (27) est en outre configurée pour commander au module de commutation (2512) de la boucle de blindage (251) du second circuit de blindage électromagnétique (25b) de relier le second circuit d'appariement (2511) de la boucle de blindage (251) du second circuit de blindage électromagnétique (25b) au second module d'antenne (28b), commander au module de commutation (2512) de la boucle de blindage (251) du second circuit de blindage électromagnétique (25b) de relier le second circuit d'appariement (2511) de la boucle de blindage (251) du second circuit de blindage électromagnétique (25b) au second dispositif d'écriture/de lecture d'informations (23b), et commander au second dispositif d'écriture/de lecture d'informations (23b) de fournir un signal de blindage au second circuit de blindage électromagnétique (25b) ; ou
lorsque le second dispositif d'écriture/de lecture d'informations (23b) transmet le second champ électromagnétique au moyen du second circuit de transmission de signal (26b) et du second module d'antenne (28b), l'unité de commande (27) est en outre configurée pour commander au module de commutation (2512) de la boucle de blindage (251) du premier circuit de blindage électromagnétique (25a) de relier le second circuit d'appariement (2511) de la boucle de blindage (251) du premier circuit de blindage électromagnétique (25a) au premier module d'antenne (28a), commander au module de commutation (2512) de la boucle de blindage (251) du premier circuit de blindage électromagnétique (25a) de relier le second circuit d'appariement (2511) de la boucle de blindage (251) du premier circuit de blindage électromagnétique (25a) au premier dispositif d'écriture/de lecture d'informations (23a), et commander au premier dispositif d'écriture/de lecture d'informations (23a) de fournir un signal de blindage au premier circuit de blindage électromagnétique (25a).

12. Analyseur d'échantillons (100) selon l'une quelconque des revendications 6 à 11, **caractérisé en ce que** chaque première antenne (281) est en outre configurée pour transmettre le contre-champ électromagnétique, où le premier circuit de blindage électromagnétique (25a) peut être relié à la première antenne (281) du premier module d'antenne (28a) pour générer le premier contre-champ électromagnétique, et le second circuit de blindage électromagnétique (25b) peut être relié à la première antenne (281) du second module d'antenne (28b) pour générer le second contre-champ électromagnétique ; et
lorsque le premier dispositif d'écriture/de lecture d'informations (23a) transmet le premier champ électromagnétique au moyen du premier circuit de transmission de signal (26a) et du premier module d'antenne (28a), l'unité de commande (27) est en outre configurée pour commander au second circuit de blindage électromagnétique (25b) de se relier à la première antenne (281) du second module d'antenne (28b) pour générer le second contre-champ électromagnétique ; ou
lorsque le second dispositif d'écriture/de lecture d'informations (23b) transmet le second champ électromagnétique au moyen du second circuit de transmission de signal (26b) et du second module d'antenne (28b), l'unité de commande (27) est en outre configurée pour commander au premier circuit de blindage électromagnétique (25a) de se relier à la première antenne (281) du premier module d'antenne (28a) pour générer le premier contre-champ électromagnétique.

13. Analyseur d'échantillons (100) selon l'une quelconque des revendications 6 à 11, **caractérisé en ce que** chaque module d'antenne (28) est en outre doté d'une seconde antenne (282), où le premier circuit de blindage électromagnétique (25a) peut être relié à la seconde antenne (282) du premier module d'antenne (28a) pour générer le premier contre-champ électromagnétique, et le second circuit de blindage électromagnétique (25b) peut être relié à la seconde antenne (282) du second module d'antenne (28b) pour générer le second contre-champ électromagnétique ; et
lorsque le premier dispositif d'écriture/de lecture d'informations (23a) transmet le premier champ électromagnétique au moyen du premier circuit de transmission de signal (26a) et de la première antenne (281) du premier module d'antenne (28a), l'unité de commande (27) est en outre configurée pour commander au second circuit de blindage électromagnétique (25b) de se relier à la seconde antenne (282) du second module d'antenne (28b) pour générer le second contre-champ électromagnétique ; ou
lorsque le second dispositif d'écriture/de lecture d'informations (23b) transmet le second champ électromagnétique au moyen du second circuit de transmission de signal (26b) et de la première antenne (281) du second module d'antenne (28b), l'unité de commande (27) est en outre configurée pour commander au premier circuit de blindage électromagnétique (25a) de se relier à la seconde antenne (282) du premier module d'antenne (28a) pour générer le premier contre-champ électromagnétique.

14. Procédé de lecture d'informations sur les réactifs, appliqué à un analyseur d'échantillons selon l'une quelconque des revendications 2 à 13, **caractérisé en ce que** le procédé comprend les étapes consistant à :
commander au premier dispositif d'écriture/de lecture d'informations de transmettre le premier champ électromagnétique au moyen du premier circuit de transmission de signal et du premier module d'antenne au composant de stockage d'informations du contenant de réactif dans la première position de réactif, de sorte que le premier dispositif d'écriture/de lecture d'informations réalise une interaction de signal avec le composant de stockage d'informations du contenant de réactif dans la première position de réactif ; et commander au second circuit de blindage électromagnétique de se relier au second module d'antenne pour générer le second contre-champ électromagnétique, de sorte à affaiblir un champ électromagnétique transmis par le premier champ électromagnétique à la seconde position de réactif ;
commander au second dispositif d'écriture/de lecture d'informations de transmettre le second champ électromagnétique au moyen du second circuit de transmission de signal et du second module d'antenne au composant de stockage d'informations du contenant de réactif dans la seconde position de réactif, de sorte que le second dispositif d'écriture/de lecture d'informations réalise une interaction de signal avec le composant de stockage d'informations du contenant de réactif dans la seconde position de réactif ; et commander au premier circuit de blindage électromagnétique de se relier au premier module d'antenne pour générer le premier contre-champ électromagnétique, de sorte à affaiblir un champ électromagnétique transmis par le second champ électromagnétique à la première position de réactif.

15. Procédé de lecture d'informations sur les réactifs selon la revendication 14, **caractérisé en ce que** le fait de commander au second circuit de blindage électromagnétique de se relier au second module d'antenne pour générer le second contre-champ électromagnétique, de sorte à affaiblir un champ électromagnétique transmis par le premier champ électromagnétique à la seconde position de réactif comprend :
le fait de régler un courant du second circuit de blindage électromagnétique, de sorte qu'un sens du courant du second circuit de blindage électromagnétique est opposé à celui d'un courant du premier circuit de transmission de signal ; ou
le fait de commander au premier circuit de blindage électromagnétique de se relier au premier module d'antenne pour générer le premier contre-champ électromagnétique, de sorte à affaiblir un champ électromagnétique transmis par le second champ électromagnétique à la première position de réactif comprend :
le fait de régler un courant du premier circuit de blindage électromagnétique, de sorte qu'un sens du courant du premier circuit de blindage électromagnétique est opposé à celui d'un courant du second circuit de transmission de signal.
